# EUROPEAN PATENT SPECIFICATION

(11) **EP 3 071 196 B1**
(45) Date of publication and mention of the grant of the patent: **27.03.2019**
(21) Application number: 14824959.2
(22) Date of filing: 19.11.2014
(51) Int. Cl.: A61K 31/166, A61K 31/167, A61P 3/00, A61P 21/00

(54) **HDAC INHIBITORS FOR SUPPRESSING CANCER-RELATED CACHEXIA**
HDAC-INHIBITOREN ZUR UNTERDRÜCKUNG VON KREBSKACHEXIE
INHIBITEURS D'HDAC POUR SUPPRIMER LA CACHEXIE ASSOCIÉE AU CANCER

(30) Priority: 20.11.2013 US 201361906738 P
(43) Date of publication of application: 28.09.2016
(73) Proprietor: Ohio State Innovation Foundation, Columbus, OH 43201 (US)
(72) Inventor: CHEN, Ching-Shih, Upper Arlington, Ohio 43221 (US); BEKAII-SAAB, Tanios, Upper Arlington, Ohio 43221 (US); GUTTRIDGE, Denis, Upper Arlington, Ohio 43220 (US); MARCUCCI, Guido, Azusa, CA 91702 (US); KULP, Samuel, Hilliard, Ohio 43026 (US); TSENG, Yu-Chou, Columbus, Ohio 43210 (US)
(74) Representative: Murgitroyd & Company
(86) International application number: PCT/US2014/066435
(87) International publication number: WO 2015/077353

(56) References cited:
- WO-A1-2013/080120
- WO-A2-2007/147868
- US-A1- 2006 229 237
- LU YEN-SHEN ET AL: "Efficacy of a novel histone deacetylase inhibitor in murine models of hepatocellular carcinoma.", HEPATOLOGY (BALTIMORE, MD.) OCT 2007, vol. 46, no. 4, October 2007 (2007-10), pages 1119-1130, XP002735991, ISSN: 0270-9139
- Tseng: "Abstract 5540: Preclinical investigation of the novel histone deacetylase (HDAC) inhibitor AR-42 in the treatment of cancer-induced cachexia", , 9 April 2014 (2014-04-09), XP002735992, Retrieved from the Internet: URL:http://cancerres.aacrjournals.org/cont ent/74/19_Supplement/5540.short [retrieved on 2015-02-12]
- KIM HYUN-JUNG ET AL: "Histone deacetylase inhibitors: molecular mechanisms of action and clinical trials as anti-cancer drugs.", AMERICAN JOURNAL OF TRANSLATIONAL RESEARCH FEB 2011, vol. 3, no. 2, February 2011 (2011-02), pages 166-179, XP002735993, ISSN: 1943-8141
- BEHARRY ADAM W ET AL: "HDAC1 activates FoxO and is both sufficient and required for skeletal muscle atrophy", JOURNAL OF CELL SCIENCE, vol. 127, no. 7, April 2014 (2014-04), pages 1441-1453, XP002735994,
- LEE SUNG YONG ET AL: "Histone deacetylase inhibitor AR-42 enhances E7-specific CD8<+> T cell-mediated antitumor immunity induced by therapeutic HPV DNA vaccination.", JOURNAL OF MOLECULAR MEDICINE (BERLIN, GERMANY) OCT 2013, vol. 91, no. 10, October 2013 (2013-10), pages 1221-1231, XP002739179, ISSN: 1432-1440

## Description

### PRIORITY CLAIM

This application claims priority to U.S. Provisional Patent Application Serial No. 61/906,738 filed on November 20, 2013.

### BACKGROUND

Cancer-related cachexia is a debilitating condition associated with loss of muscle mass, fatigue, weakness, and loss of appetite in cancer patients. Cachexia is also associated with severe clinical consequences including muscle weakness which can result in ambulation difficulties, and pulmonary complications. Cachexia is a significant contributing factor in the death of cancer patients.

Cachexia is characterized by depletion of skeletal muscle mass that is not reversed by conventional nutritional support, leading to pronounced weight loss that severely impacts patient morbidity and mortality. It occurs in more than 80% of patients with gastric, pancreatic, and esophageal cancer; 70% of those with head and neck cancer; and approximately 60% of patients with lung, colorectal, and prostate cancer. See., e.g., Muscle (2012) 3, 245-51. Despite cachexia's impact on mortality among cancer patients, no effective therapies have been developed to prevent or impede the progression of cachexia. For example, more than 85% of pancreatic cancer patients, including early stage patients, are estimated to lose an average of 14% of their pre-illness weight. See, e.g., BMC Cancer. 2010 Jul 8;10:363. Cachectic pancreatic cancer patients are often weak and fatigued, and have a lower tolerance to therapy and more adverse outcomes to surgery. Consequently, cachexia is the main driver for mortality in pancreas cancer. Sadly, the 5-year survival rate for pancreatic cancer remains at 6% for the last four decades, which is the lowest among all malignancies.

With the advent of new tools to identify cachectic factors and their effects on skeletal muscle, the field of cachexia has recently made significant advances in understanding the underlying mechanisms that regulate muscle atrophy in cancer and other chronic illnesses. As a result, we now have an appreciation for how cytokines and systemic inflammation regulate muscle atrophy by acting on key signaling pathways that operate from inside the myofiber. However, translating these discoveries into effective therapies has been challenging, and, prior to the aspects described herein, an effective treatment for cachexia has been lacking.

Skeletal muscle mass is regulated, in part, by the relative rate of protein synthesis versus protein degradation. Alamdari, N, et al., Acetylation and deacetylation - novel factors in muscle wasting, Metabolism. Jan 2013; 62(1): 1-11. Loss of skeletal muscle mass occurs when the rate of protein degradation is greater than protein synthesis. Protein acetylation and deacetylation modify transcription factors and gene transcription which may influence muscle mass by rendering proteins more or less susceptible to degradation. Histone acetylases (HATs) and histone deacetylases (HDACs) play a role in regulating protein acetylation and deacetylation.

However, the effects of these molecules on muscle wasting and cachexia have proven to be contradictory - evidence suggests, for example, that use of HDAC inhibitors (e.g., Trichostatin A (TSA)) results in hyperacetylation which can increase protein degradation leading to increased muscle wasting and cachexia. Contradictory results were found by Narver et. al., (Sustained improvement of spinal muscular atrophy mice treated with trichostatin A plus nutrition. Ann Neurol. 2008;64:465-70) however, these results have been questioned because treatment with TSA was also accompanied by aggressive nutritional support. Alamdari et al., at 5. Thus, HDAC inhibitors were thought to increase rather than decrease cachexia or their use produced conflicting and contradictory results.

The development and progression of cancer cachexia is caused by complex, multifactorial pathophysiological responses to tumors in muscle tissues. To date, no FDA-approved therapies are available to prevent or hamper the progression of muscle wasting in cachectic patients. To date, several investigational drugs, which target different aspects of cachexia pathogenesis, have undergone human trials, however, with different clinical outcomes. For example, while Novartis's BYM38 (bimagrumab), a mAb that blocks binding of myostatin and activin to type II activin receptors, received FDA breakthrough therapy designation, GTx's muscle wasting drug enobosarm, a selective androgen receptor modulator, failed in late-stage clinical trials.

Acetylation of core histones plays an important role in the regulation of gene transcription by controlling nucleosomal packaging of DNA. Deacetylation of histones results in tight packing of nucleosomes and transcriptional repression due to limited access of transcription factors to DNA targets. Histone acetylation relaxes nucleosome structures, providing greater access for transcription factors. The balance between histone deacetylation and acetylation is modulated by the histone deacetyl-transferases (HDACs) and histone acetyl-transferases (HAT). An abnormal balance of these factors is correlated with abnormal cell growth and several forms of cancer as discussed in U.S. Patent Number 8,318,808,. HDAC inhibitors, in particular, change the balance between acetylation and deacetylation resulting in growth arrest, differentiation, and apoptosis in many tumor cell types. See, e.g., U.S. Patent Number 8,318,808.

18 HDACs have been identified in humans and are characterized as being zinc dependent or nicotinamide adenine dinucleotide (NAD) dependent (Discov Med 10(54):462-470, November 2010) and are associated with the following classes: class I (HDACs 1, 2, 3, and 8); class II (HDACs 4, 5, 6, 7, 9, and 10; class III (sirtuins 1-7 (SIRT)); and class IV (HDAC 11).

WO 2007/147868 discloses a HDAC6 inhibitor for use in a method for reducing muscle atrophy and cachexia associated with cancer.

Of particular interest herein are HDAC inhibitors described in U.S. Patent Number 8,318,808 and are based on, for example, fatty acids coupled with Zn²⁺-chelating motifs through aromatic Ω-amino acid linkers. In various aspects, the HDAC inhibitors may have the formula: 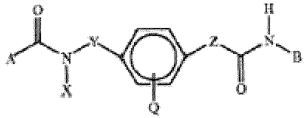 wherein X is chosen from H and CH3; Y is (CH2)n wherein n is 0-2; Z is chosen from (CH2)m wherein m is 0-3 and (CH)2; A is a hydrocarbyl group; B is o-aminophenyl or hydroxyl group; and Q is a halogen, hydrogen, or methyl. One HDAC inhibitor in particular (N-hydroxy-4-(3-methyl-2-phenyl-butyrylamino)-benzamide) is also known as AR-42. The structure of AR-42 is as follows:

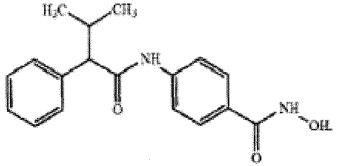

AR-42 is a broad-spectrum deacetylase inhibitor of both histone and non-histone proteins with demonstrated greater potency and activity in solid tumors and hematological malignancies when compared to vorinostat (i.e., SAHA). See, e.g., Lu YS, et al., Efficacy of a novel histone deacetylase inhibitor in murine models of hepatocellular carcinoma, Hepatology. 2007 Oct;46(4):1119-30; Kulp SK, et al., Antitumor effects of a novel phenylbutyrate-based histone deacetylase inhibitor, (S)-HDAC-42, in prostate cancer, Clin Cancer Res. 2006 Sep 1; 12(17):5199-206.

AR-42 may also possess additional histone-independent mechanisms which contribute to its therapeutic profile. See, e.g., Chen MC, et al., Novel mechanism by which histone deacetylase inhibitors facilitate topoisomerase IIα degradation in hepatocellular carcinoma cells, Hepatology. 2011 Jan;53(1):148-59; Chen CS, et al., Histone acetylation-independent effect of histone deacetylase inhibitors on Akt through the reshuffling of protein phosphatase 1 complexes, J Biol Chem. 2005 Nov 18;280(46):38879-87; Yoo CB, et al., Epigenetic therapy of cancer: past, present and future, Nat Rev Drug Discov. 2006 Jan;5(1):37-50.

AR-42 has a demonstrated inhibitory effect in tumors including, but not limited to, breast, prostate, ovarian, blood cell (e.g., lymphoma, myeloma, and leukemia), liver, and brain. See, e.g., Mims A, et. al., Increased anti-leukemic activity of decitabine via AR-42-induced upregulation of miR-29b: a novel epigenetic-targeting approach in acute myeloid leukemia, Leukemia. 2012 Nov 26. doi: 10.1038/leu.2012.342. [Epub ahead of print]; Burns SS, et al., Histone deacetylase inhibitor AR-42 differentially affects cell-cycle transit in meningeal and meningioma cells, potently inhibiting NF2-deficient meningioma growth, Cancer Res. 2013 Jan 15;73(2):792-803; Lu YS, et. al., Radiosensitizing effect of a phenylbutyrate-derived histone deacetylase inhibitor in hepatocellular carcinoma, Int J Radiat Oncol Biol Phys. 2012 Jun 1;83(2); Zimmerman B, et. al., Efficacy of novel histone deacetylase inhibitor, AR42, in a mouse model of, human T-lymphotropic virus type 1 adult T cell lymphoma, Leuk Res. 2011 Nov;35(11):1491-7; Zhang S, et al., The novel histone deacetylase inhibitor, AR-42, inhibits gp130/Stat3 pathway and induces apoptosis and cell cycle arrest in multiple myeloma cells, Int J Cancer. 2011 Jul 1;129(1):204-13.

### SUMMARY

Aspects described herein provide methods of suppressing cachexia in a mammal with cancer comprising administering HDAC class 1 and 2b inhibitor AR-42 to said mammal. One aspect provides a method of suppressing cachexia in a mammal with cancer by administering the HDAC class 1 and 2b inhibitor AR-42 to said mammal in an amount effective to substantially maintain the mammal's weight compared to a mammal that does not receive the HDAC class 1 and 2b inhibitor AR-42. In another aspect, the HDAC class 1 and 2b inhibitorAR-42is administered to a mammal with cancer in an amount effective to substantially maintain at least about 90% of said mammal's weight over a period of time of at least fifteen days. In yet another aspect, the mammal with cancer has at least one tumor and the tumor volume is not reduced by more than 6% during about the first fifteen days following treatment with AR-42.

Other aspects described herein provide methods of suppressing cachexia by administering AR-42 to a mammal with cancer wherein the expression of multiple mediators of muscle atrophy (e.g., pro-cachexia drivers such as IL-6, IL-6Rα, LIF, MuRF1, Atrogin-I) in cancer cachexia is reduced compared to a mammal having cancer that is not treated with AR-42.

Further aspects provide methods of suppressing cachexia by administering AR-42 to a mammal with cancer wherein cachexia-induced loss of adipose tissue and reduction in skeletal muscle fiber size is substantially restored compared to a mammal that does not receive AR-42.

As disclosed herein, AR-42 shows *in vivo* efficacy in suppressing, reducing, or blocking muscle wasting and prolonging survival in animal models of cancer cachexia. In addition, the effect of AR-42 on cancer-related cachexia is independent of AR-42's effects on reducing tumor load.

### FIGURES

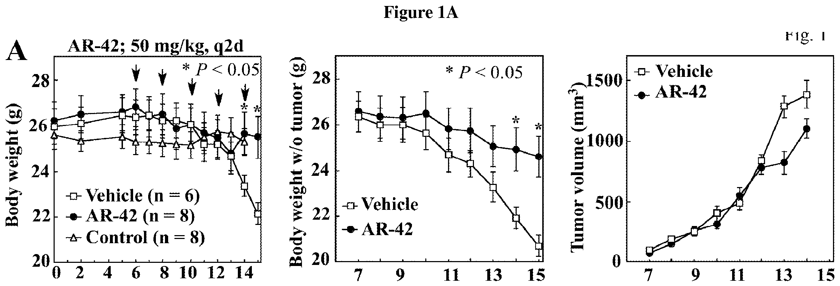

Figure 1A shows exemplary suppression of cancer-induced cachexia in C-26 tumor-bearing mice and depicts changes in total weight (*left*, tumor included) and body weight (*center*, tumor excluded) during the 15-day study in vehicle-treated tumor-free mice (Control) versus tumor-bearing mice treated with vehicle (Vehicle) or oral AR-42 at 50 mg/kg every other day (AR-42). Arrows indicate the times of AR-42 treatment. *Right*, lack of suppressive effect of AR-42 on tumor growth in C-26 tumor-bearing mice. Data are presented as means ± S.D. *P* values: a, 0.045; b, 0.0027; c, 0.049; d, 0.0048;

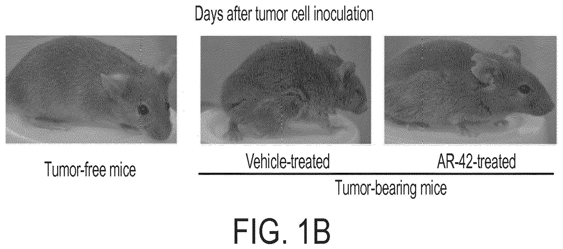

Figure 1B shows photographs of representative mice with tumor burdens from each group depicting the therapeutic effect of AR-42 on cancer cachexia;

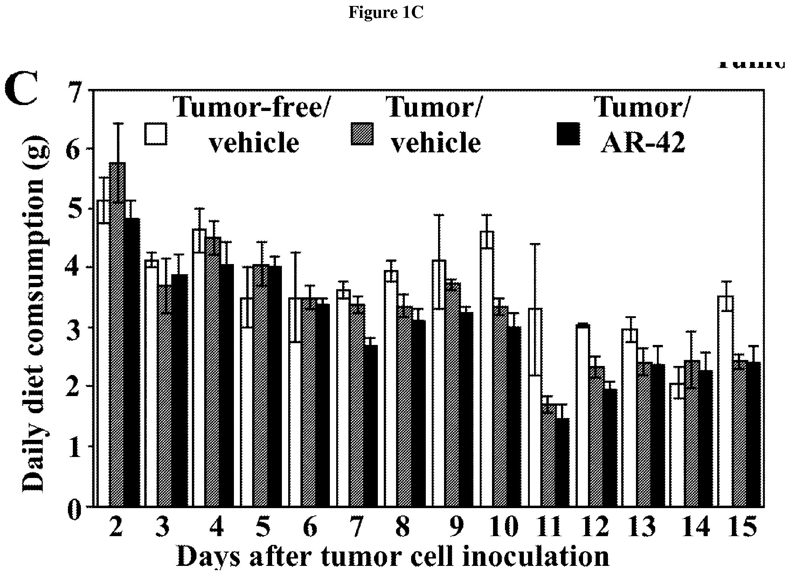

Figure 1C shows an exemplary average daily diet consumption among the three treatment groups in the course of study. Data are presented as means ± S.D. (n = 8);

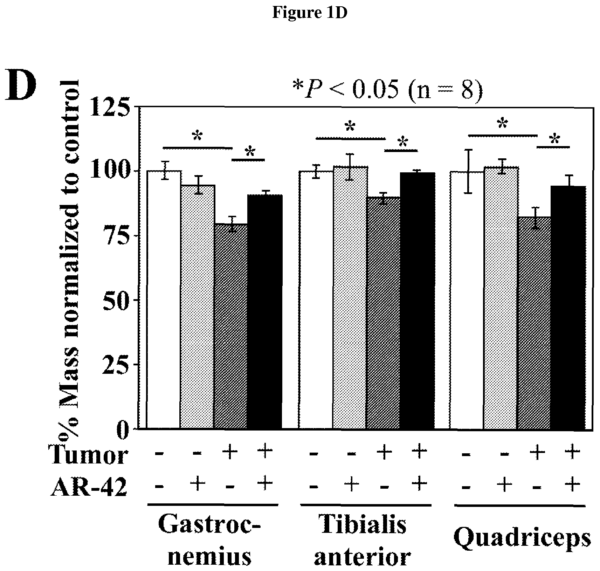

Figure 1D shows the exemplary effects of AR-42 on the weights ofhindlimb muscles, including gastrocnemius, tibialis anterior, and quadriceps (*P* values: a, <0.001; b, 0.0042; c, 0.0046) in both tumor-free and tumor-bearing mice compared to those of vehicle-treated tumor-bearing and tumor-free mice (n = 8);
Figure IE shows the exemplary effects of AR-42 on heart, adipose tissue, and spleen (*P* values: a, <0.001; b, 0.0059; c, 0.001; d, 0.009) in both tumor-free and tumor-bearing mice compared to those of vehicle-treated tumor-bearing and tumor-free mice (n = 8);

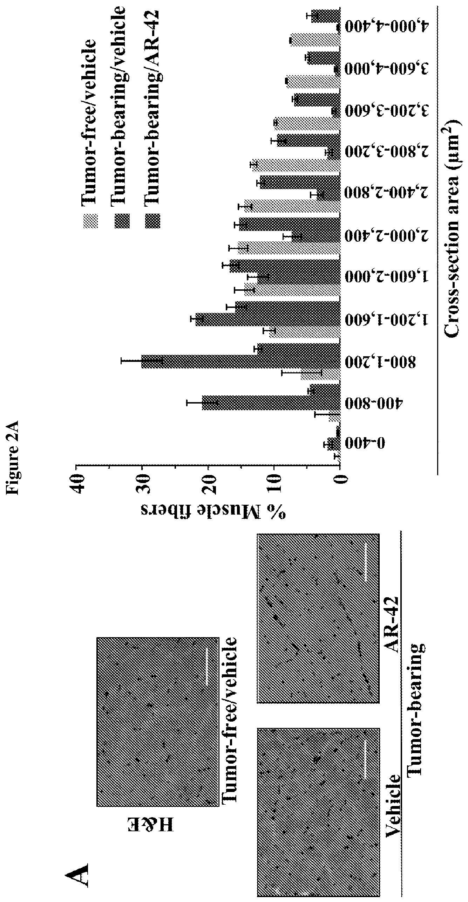

Figure 2A shows exemplary preservation of muscle fiber size in C-26 tumor bearing mice depicting on the left, photomicrographs of H&E-stained sections of gastrocnemius muscles from tumor-free control mice and tumor-bearing mice treated with vehicle or AR-42. Scale bars, 100 µm and on the right, the cross-sectional areas of muscle fibers in gastrocnemius muscles represented as a frequency histogram with a significance of (*P* < 0.001). Data are presented as means ± S.D.;

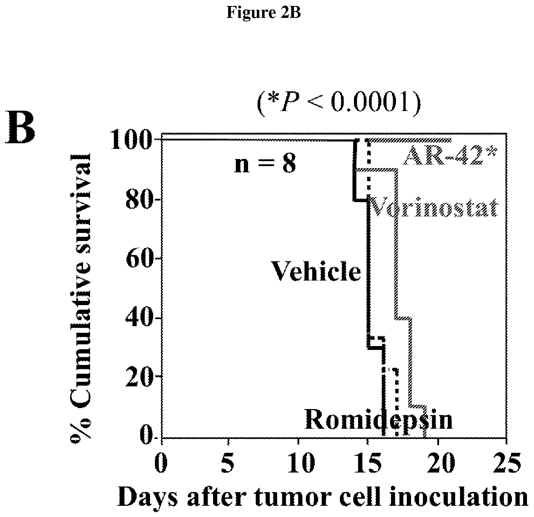

Figure 2B shows exemplary Kaplan-Meier survival curves for tumor-bearing mice treated with vehicle, vorinostat (50 mg/kg, p.o., daily), romidepsin (0.6 mg/kg, i.p., twice weekly), or AR-42 (50 mg/kg. p.o., every other day). Survival was defined as the time at which loss of body weight (tumor excluded) reached 20% of starting body weight, which served as a humane endpoint for removal from the study (*, *P* < 0.001, vehicle vs. AR-42; n = 8);

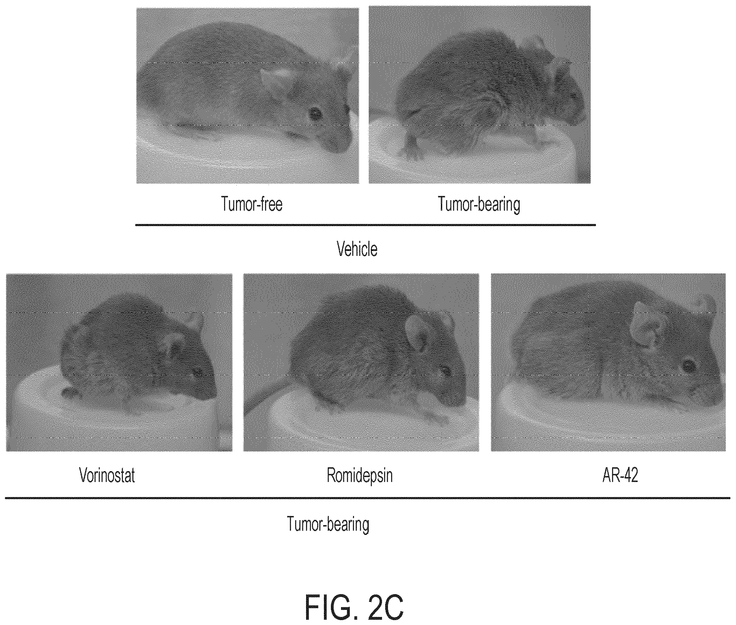

Figure 2C shows exemplary photographs of representative mice from each group depicting the therapeutic effect of AR-42 versus vorinostat and romidepsin on cancer cachexia in tumor-bearing mice, as manifested by posture, haircoat and body condition;

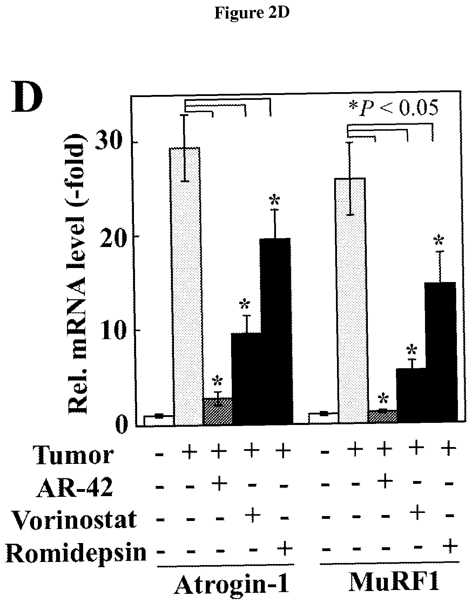

Figure 2D shows exemplary relative mRNA expression levels of Atrogin-1//MAFbx and MuRF1 in the skeletal muscles of vehicle-treated tumor-free mice (n = 6) and tumor-bearing mice treated with AR-42 (n = 8), vorinostat (n = 8), or romidepsin (n = 5) compared to that of vehicle-treated tumor-bearing mice (n = 8) at 15 days after tumor cell injection. Data are presented as means ± S.D. *P* values: a, <0.001; b, 0.016; c, 0.0063;

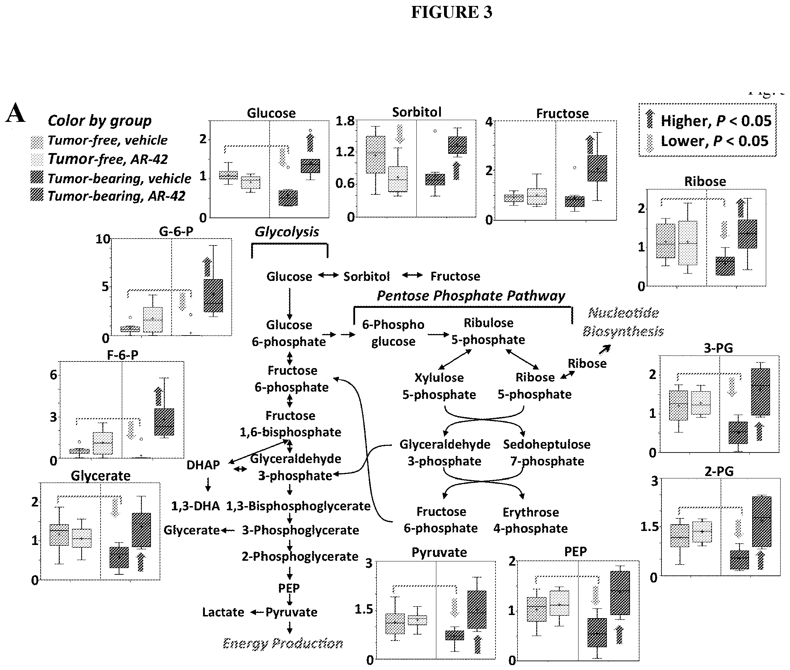

Figure 3A shows exemplary effects of AR-42 on the levels of intermediates associated with glycolysis and alternative pathways of glucose metabolism;

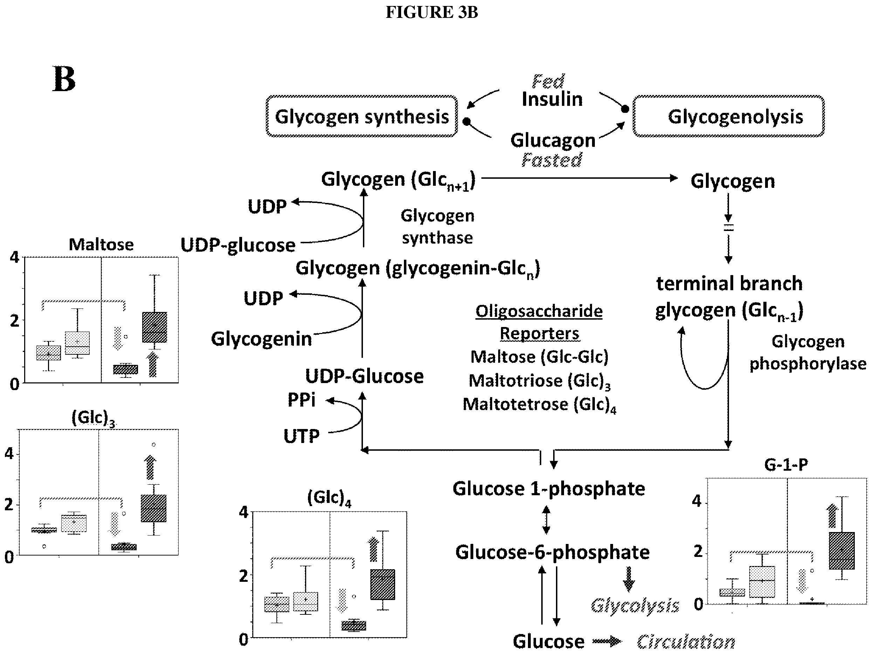

Figure 3B shows exemplary effects of AR-42 on glycogen metabolism in gastrocnemius muscles from tumor-free and tumor-bearing mice (n = 8 for each group). Tumor-bearing mice were treated with vehicle or AR-42 (50 mg/kg, p.o., every other day) beginning at day 6 post-tumor cell injection and ending at day 17. Tumor-free control mice were treated with vehicle or AR-42 in parallel. Data are presented in box-and-whisker plots. The bottom and top of the box represent the first and third quartiles, and the "+" symbol and the band inside the box denote the mean and median values, respectively. The ends of the whiskers represent the maximum and minimum values in each group;

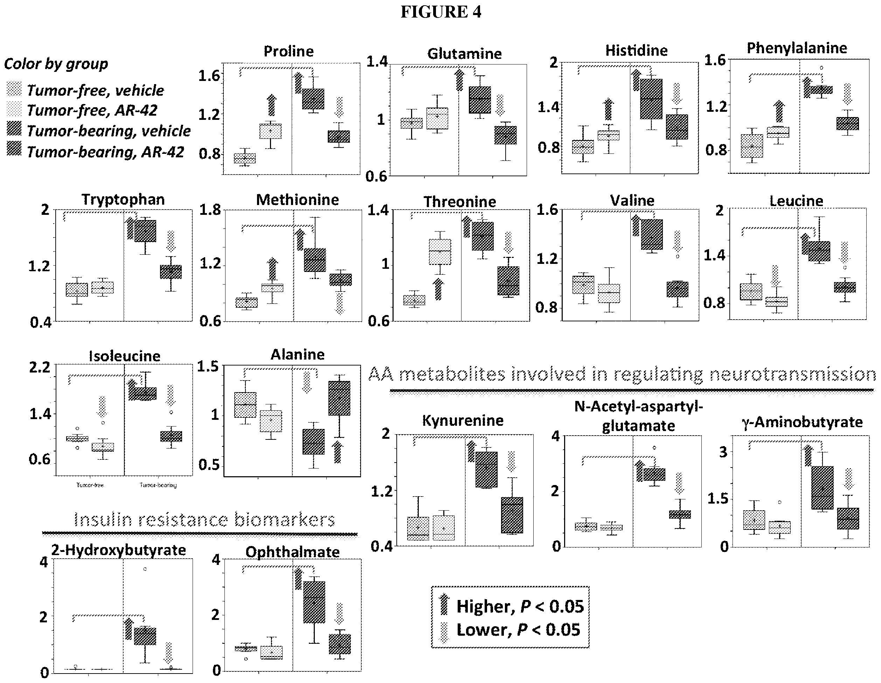

Figure 4 shows that AR-42 blocks cachexia-induced changes in the levels of free amino acids and amino acid metabolites involved in regulating neurotransmission, and biomarkers of insulin resistance in the muscles of C-26 tumor-bearing mice. Samples for analysis were generated from the experiment described with respect to Figures 3A and 3B. Data are presented in box-and-whisker plots as described with respect to Figures 3A and 3B;

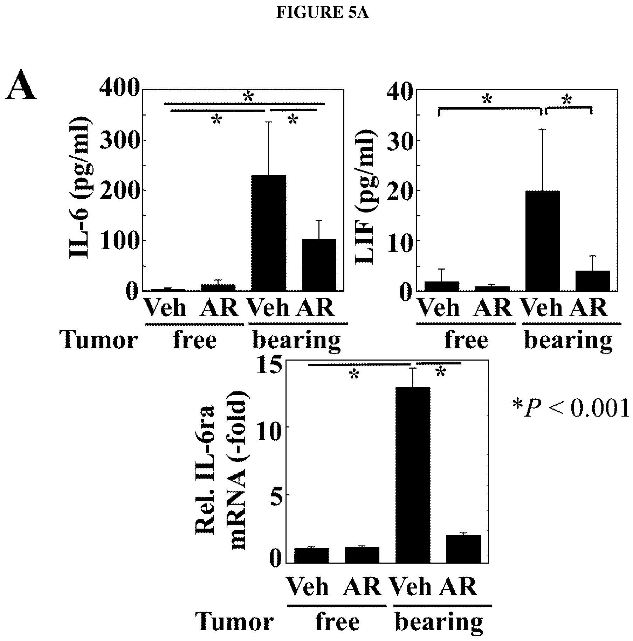

Figure 5A shows exemplary (upper) effects of AR-42 on the levels of the pro-cachexia cytokines IL-6 (left) and LIF (right) in the sera of tumor-free versus C-26 tumor-bearing mice and (lower) qPCR analysis of the effects of AR-42 on the mRNA expression of *IL-6Ra* in skeletal muscle of tumor-free versus C-26 tumor-bearing mice. Data are presented as means ± S.D. *P* values: a, <0.001; b, 0.006; c, 0.012 (n = 3). Mice were treated as described with respect to Figure 3;

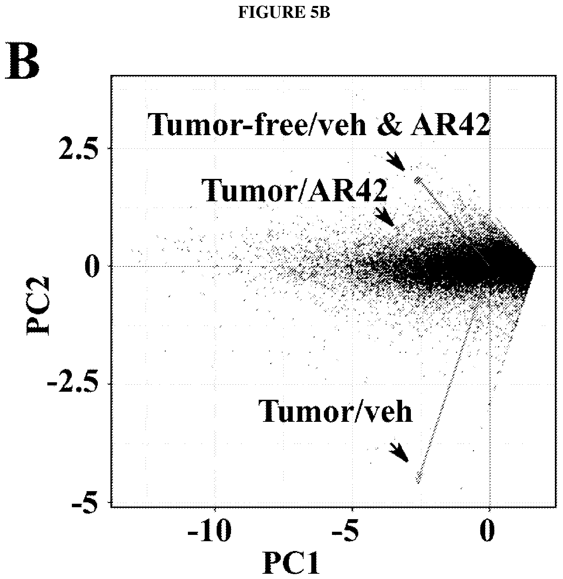

Figure 5B shows exemplary principle component analysis of RNA-seq data (left) and Venn diagram (right) showing differentially expressed genes among the four treatment groups. TF, tumor-free; T, tumor-bearing; veh, vehicle-treated; AR, AR-42-treated. Mice were treated as described with respect to Figure 3;

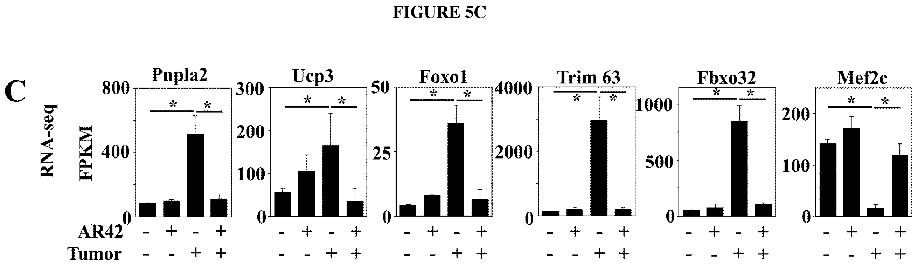

Figure 5C shows exemplary analysis of the effects of AR-42 on the transcript levels of six key pro-cachexia drivers by RNA-seq (*P* values: a, 0.024; b, 0.028; c, 0.015; d, 0.007; e, 0.024; f, 0.026; g, 0.01; h, 0.012; i, <0.001; j, 0.014; n = 3). Mice were treated as described with respect to Figure 3;

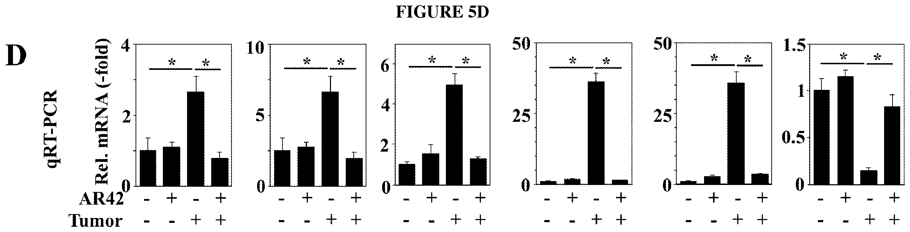

Figure 5D shows exemplary analysis of the effects of AR-42 on the transcript levels of six key pro-cachexia drivers by qPCR in skeletal muscle of mice in the four treatment groups (*, *P* < 0.001; n = 6). Data are presented as means ± S.D. Mice were treated as described with respect to Figure 3;

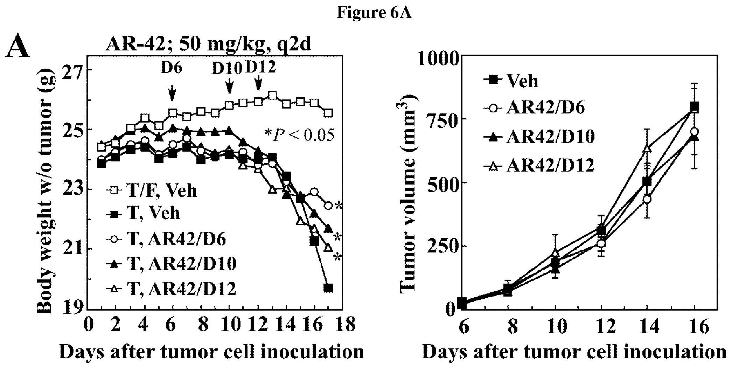

Figure 6A shows suppression of cancer cachexia in C-26 tumor-bearing mice by delaying treatment with AR-42 until late stages of tumor and cachexia. Changes in body weight (tumor excluded) in the course of 18-day study in vehicle-treated tumor-free control mice (T/F, Veh) and tumor-bearing mice treated with vehicle (T, Veh) versus those treated with oral AR-42 (shown on the left) starting at day 6 (T, AR42/D6), day 10 (T, AR42/D10), or day 12 (T, AR42/D12). *P* values: a, 0.0015; b, 0.023 (n = 8). Arrows indicate the time points for the start of AR-42 treatment. Data are presented as means. For clarity of presentation, the S.D. bars for each data point are not shown. On the right are results showing the lack of suppressive effect of AR-42 on tumor growth in C-26 tumor-bearing mice in the delayed treatment experiment. Data are presented as means ± S.D. (n = 8);

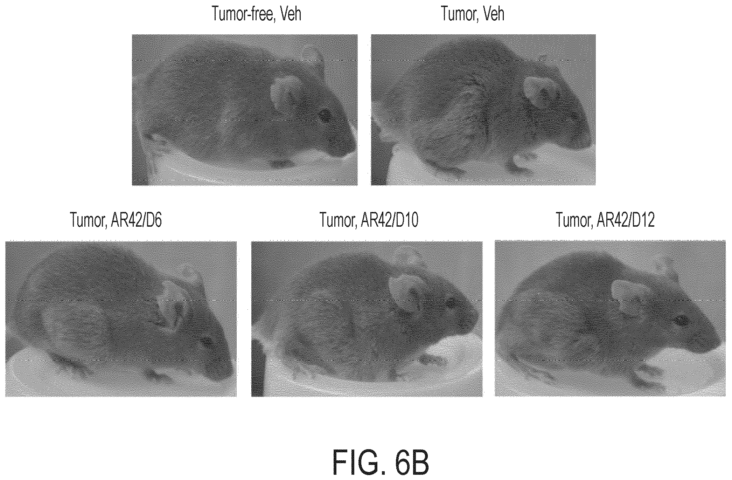

Figure 6B shows exemplary photographs depicting the therapeutic effect of AR-42 on cancer cachexia in tumor-bearing mice, despite delayed treatment, as manifested by normal posture, smooth haircoat and better body condition, despite large tumor burdens;

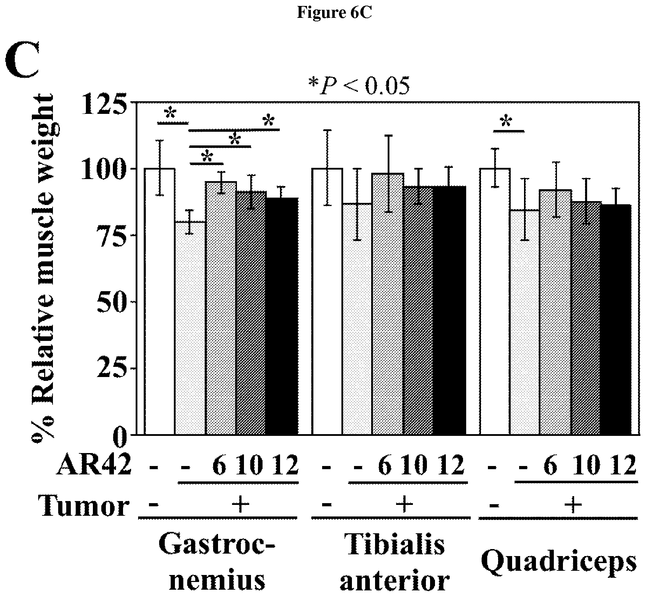

Figure 6C shows the exemplary effects of AR-42 treatment, initiated at different stages of disease progression as described in Figure 6A, on the weights of hindlimb muscles, including gastrocnemius, tibialis anterior, and quadriceps, in C-26 tumor-bearing mice. Data are presented as means ± S.D. (n = 8; *, *P* < 0.001);

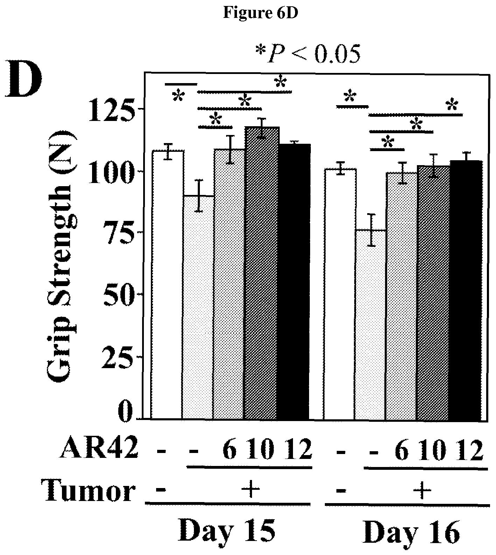

Figure 6D shows the effects of AR-42 on grip strength of tumor-bearing mice relative to the vehicle-treated tumor-free and tumor-bearing controls at day 15 and day 16. Data are presented as means ± S.D. (n = 8; *P* values: a, 0.01; b, 0.022; c, <0.001; d, 0.0019). N, Newtons;

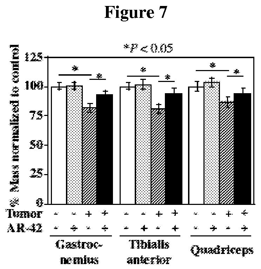

Figure 7 shows that AR-42 protects against cancer-induced muscle wasting in the LLC mouse model of cachexia. Exemplary effects of AR-42 versus vehicle on the mass of hindlimb muscles, including gastrocnemius, tibialis anterior, and quadriceps, in both tumor-free and tumor-bearing mice compared to that of vehicle-treated tumor-bearing mice are shown. Mice were treated in the same manner as described in Figure 1A, except that mice were sacrificed at day 20 after tumor cell injection. Data are presented as means ± S.D. (n = 8);

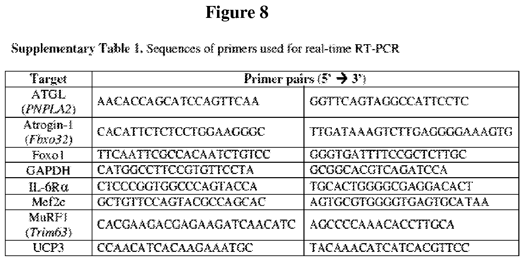

Figure 8 shows exemplary sequences of primers used for real-time RT-PCR;

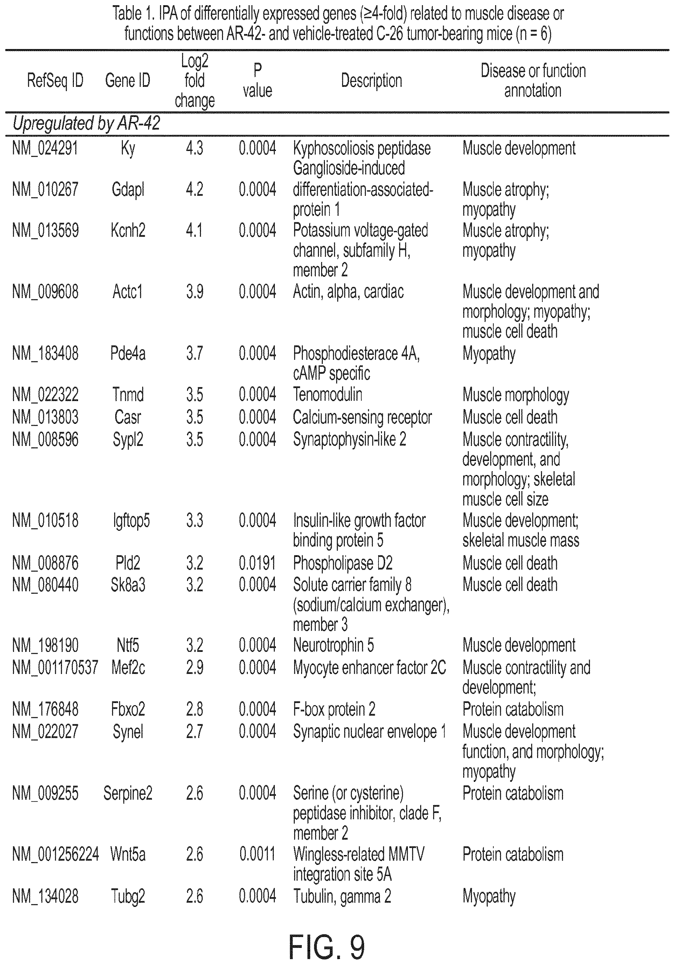

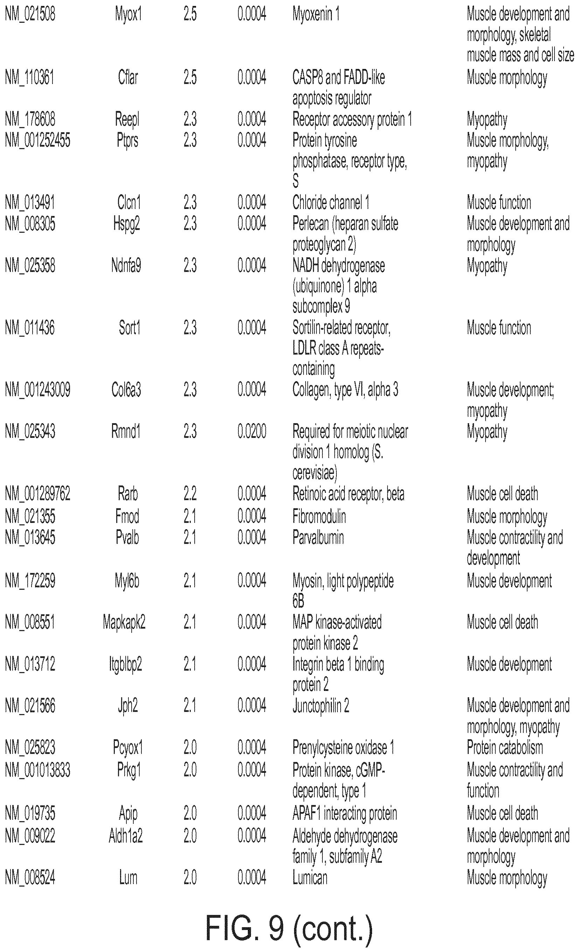

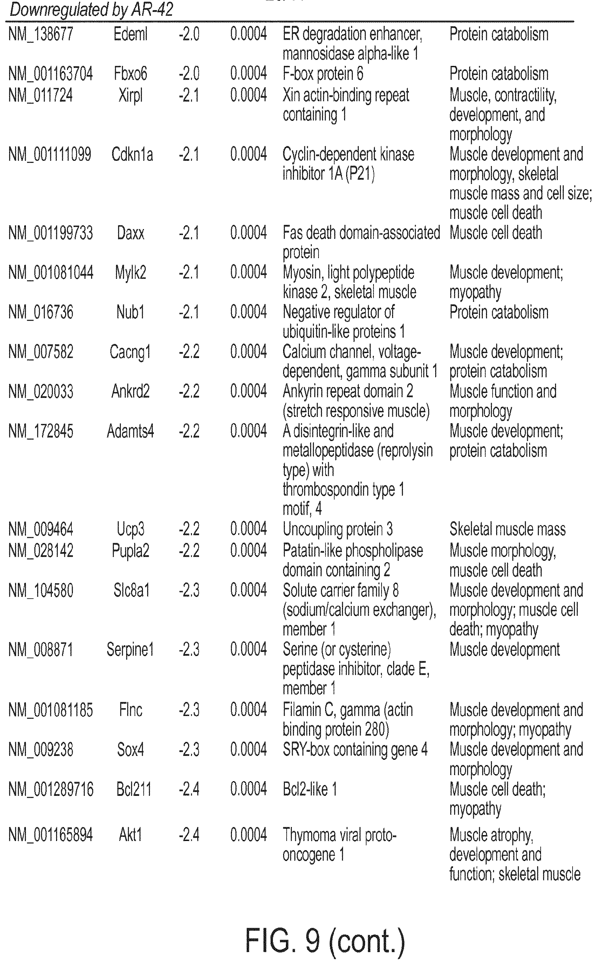

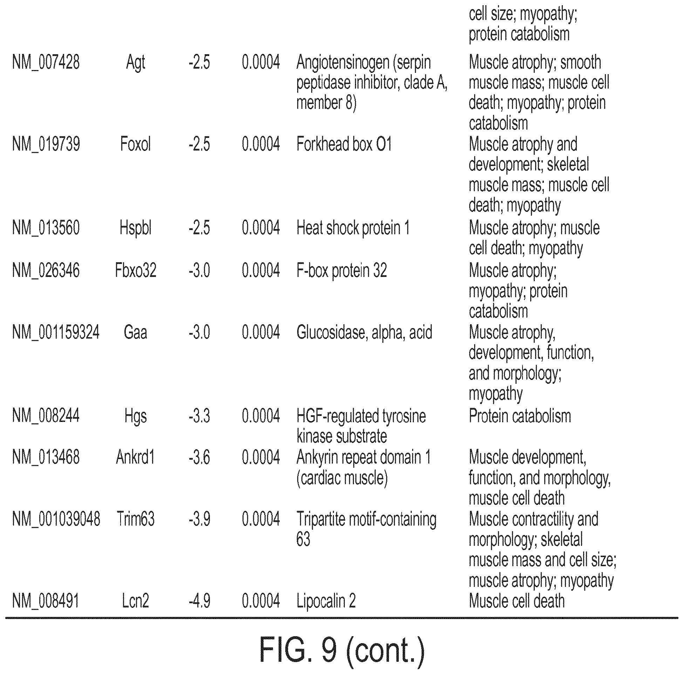

Figure 9 shows exemplary Ingenuity Pathway Analysis (IPA)(QIAGEN) of differentially expressed genes (≥4-fold) related to muscle disease or functions between AR-42- and vehicle-treated C-26 tumor-bearing mice (n = 6);

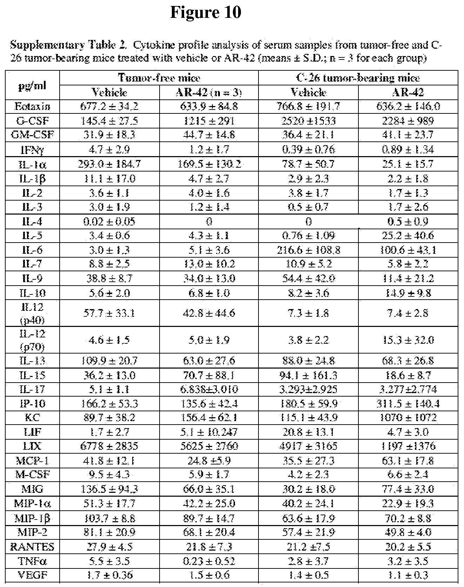

Figure 10 shows exemplary cytokine profile analysis of serum samples from tumor-free and C-26 tumor-bearing mice treated with vehicle or AR-42 (means ± S.D.; n = 3 for each group); and

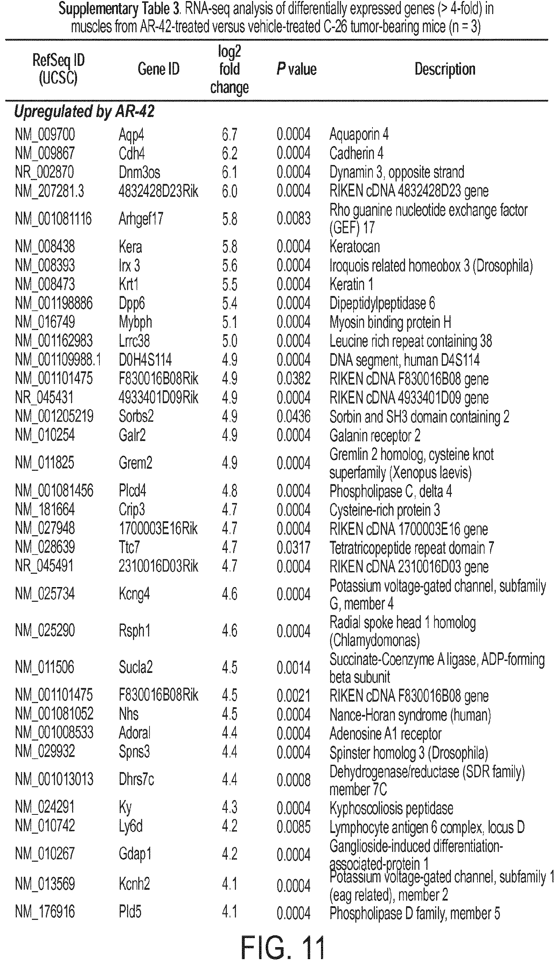

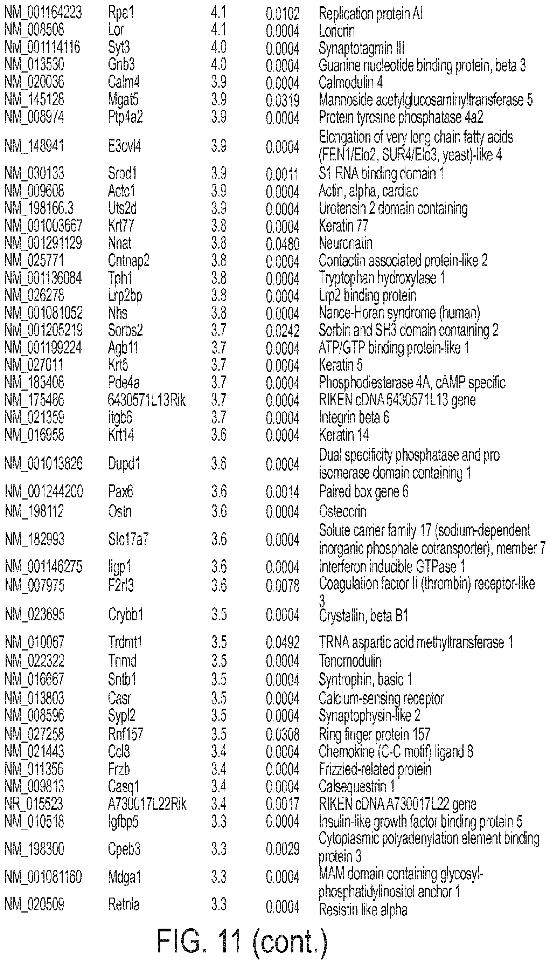

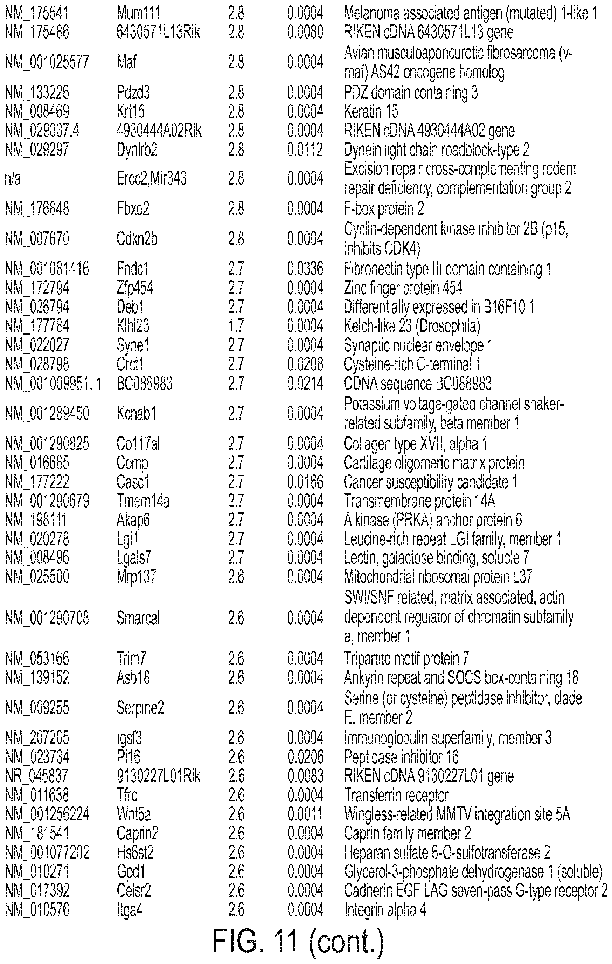

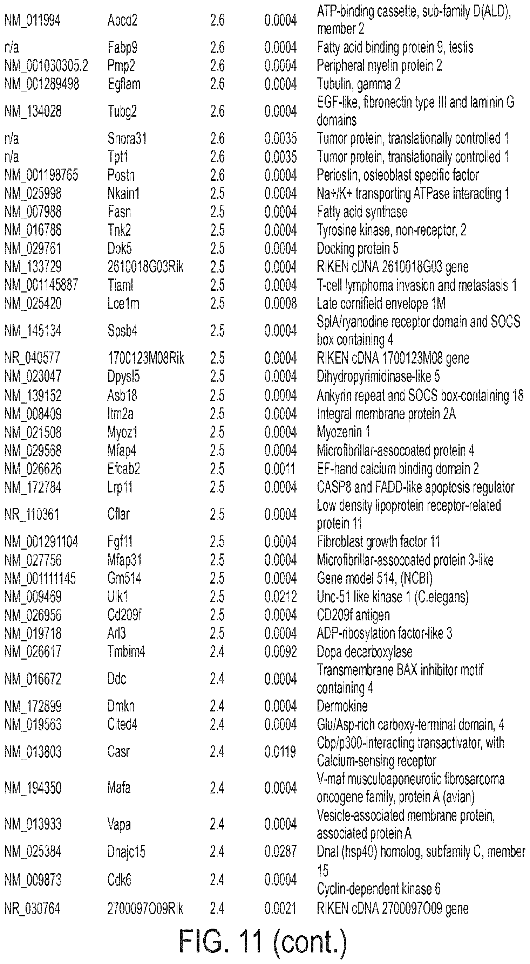

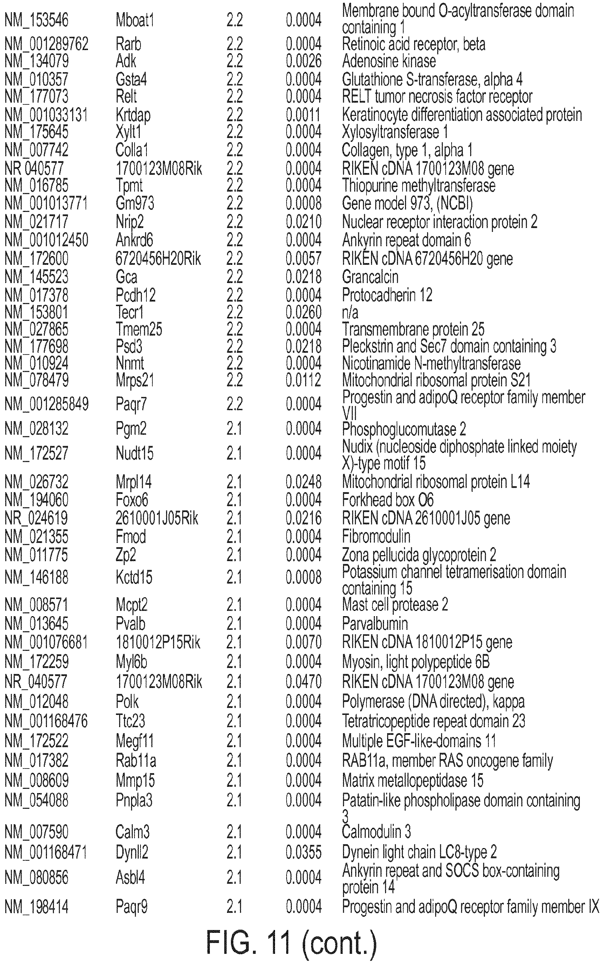

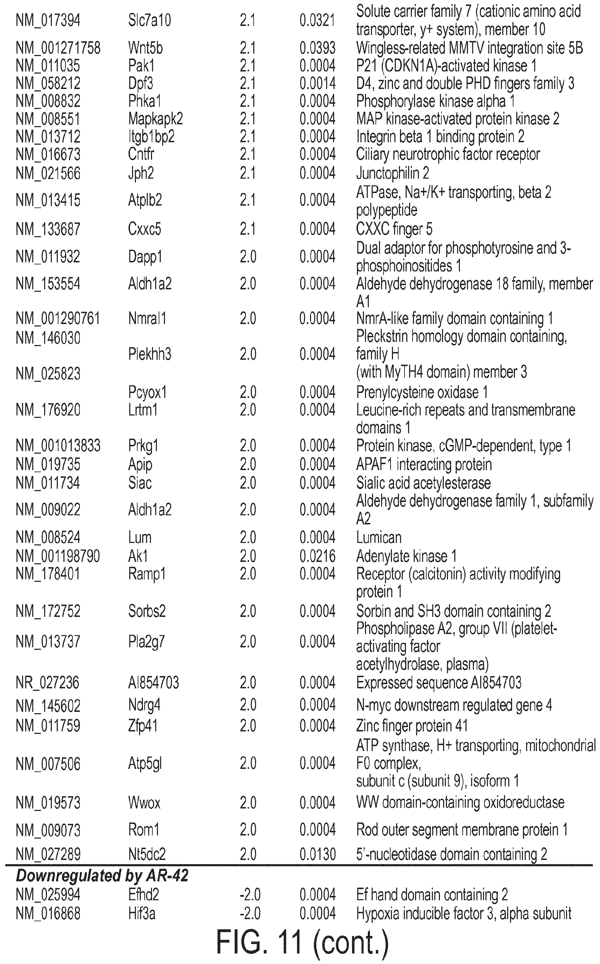

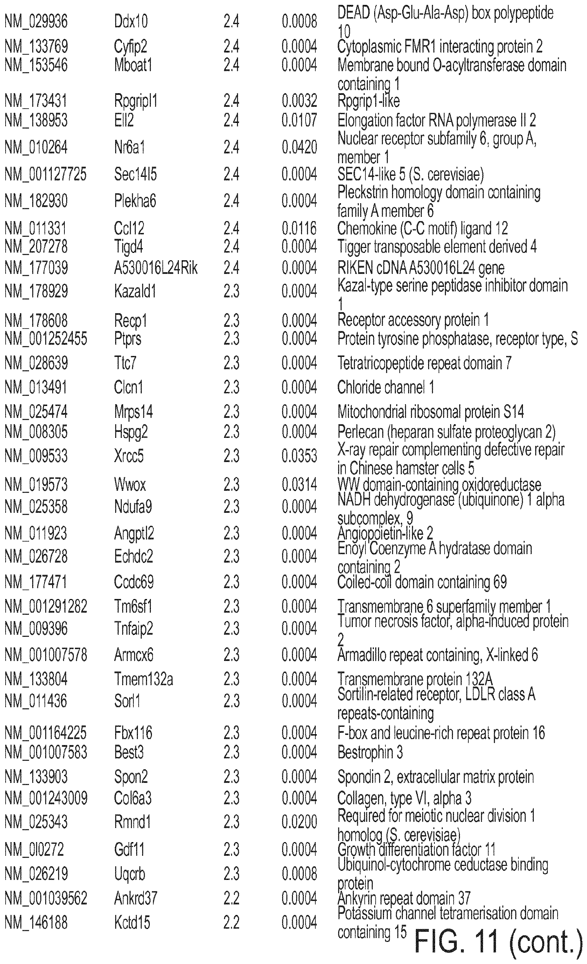

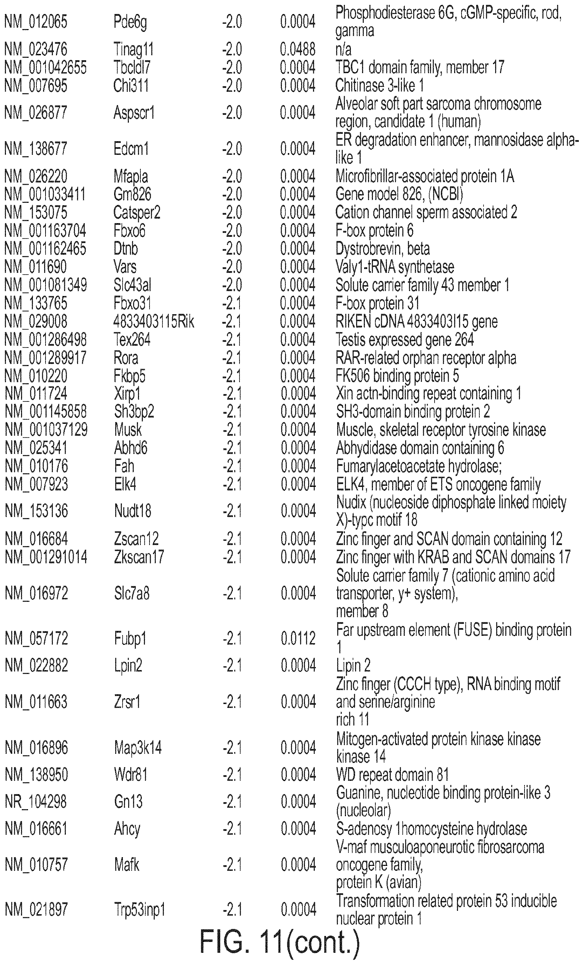

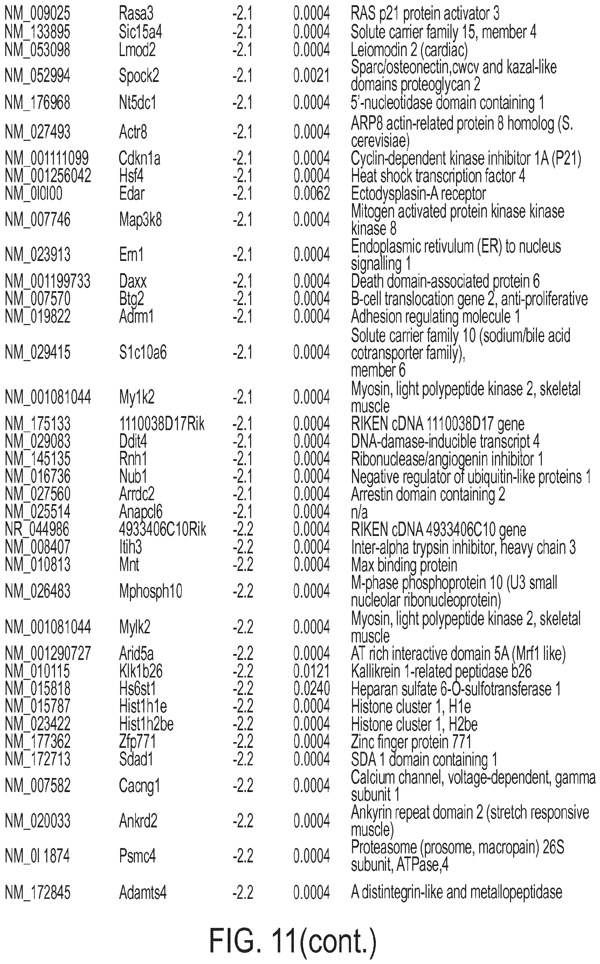

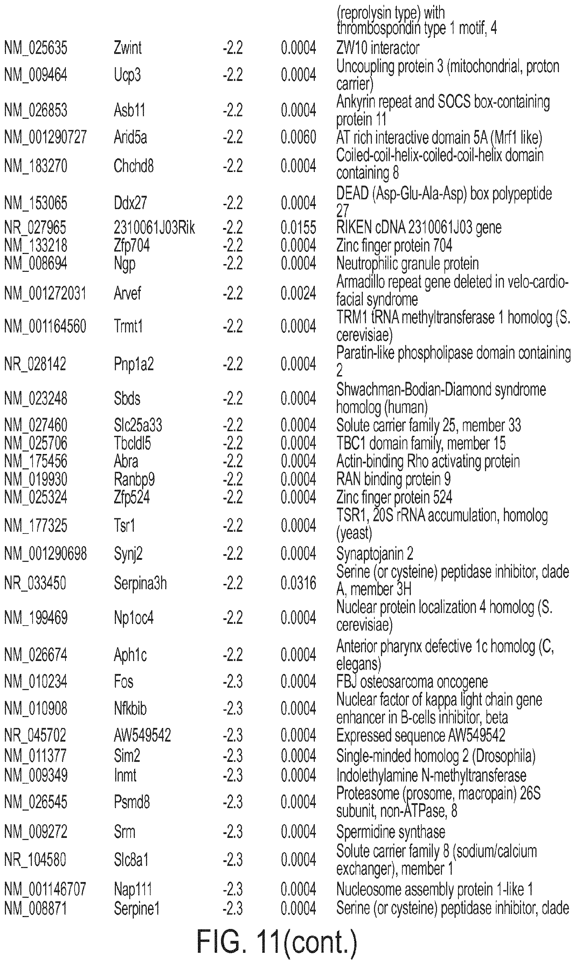

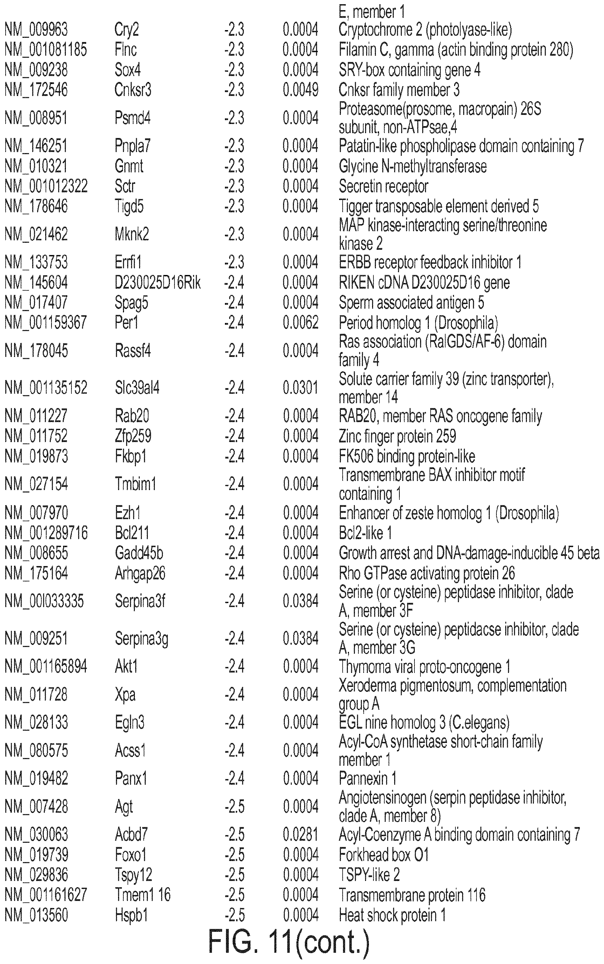

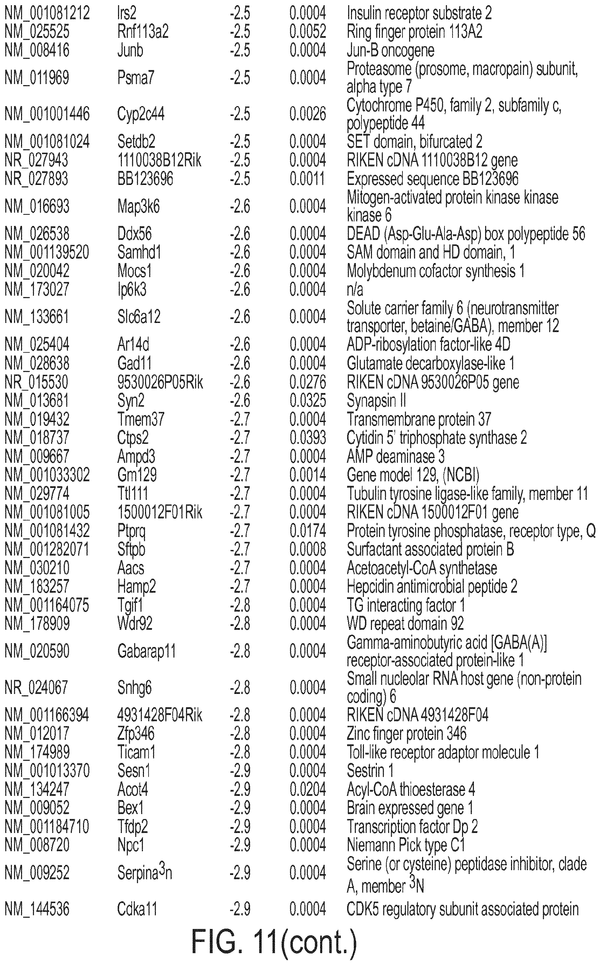

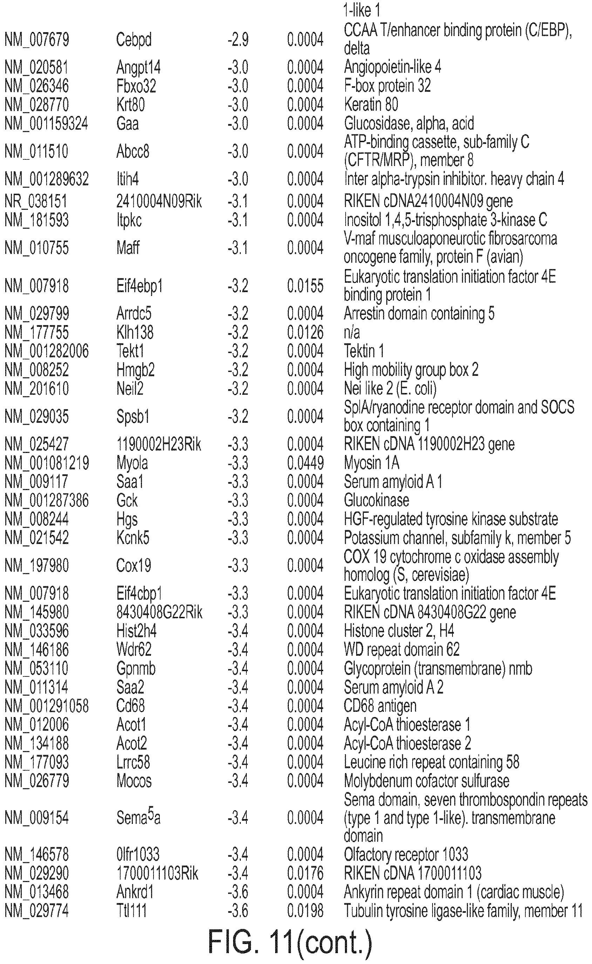

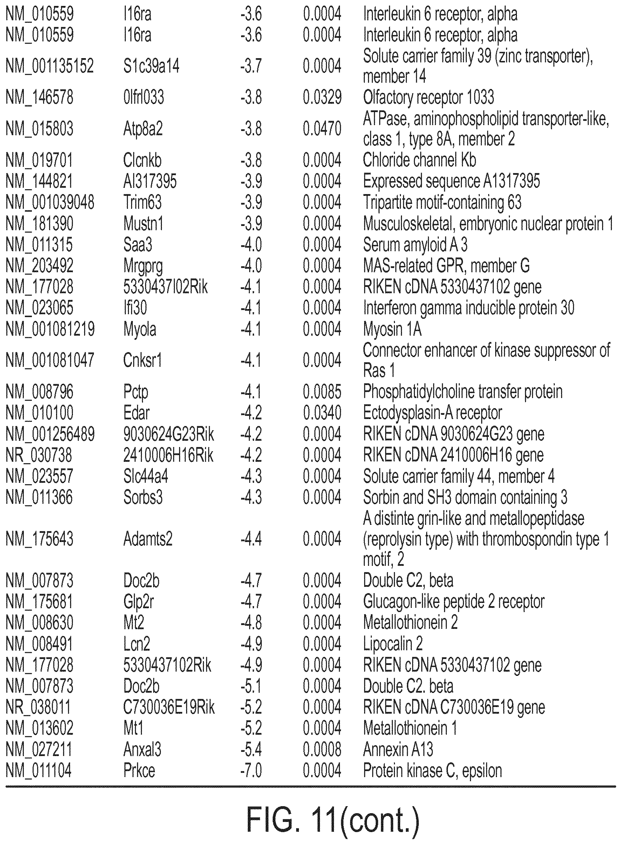

Figure 11 shows exemplary RNA-seq analysis of differentially expressed genes (≥ 4-fold) in muscles from AR-42-treated versus vehicle-treated C-26 tumor-bearing mice (n = 3).

### DETAILED DESCRIPTION

The present invention is defined in the appended claims.

Aspects described herein disclose the effects of oral AR-42 in attenuating cachexia-induced weight loss and skeletal muscle atrophy and prolonging survival in the CD2F₁ mouse colon 26 (C-26) tumor model of cancer cachexia. The mouse CD2F₁ cachexia model is described, for example, in BMC Cancer. 2010 Jul 8;10:363,. The observed anti-cachexia effect was associated with the ability of AR-42 to reprogram cell metabolism and to downregulate IL-6 levels in diseased muscle tissues to suppress muscle wasting and other cachexia-related effects independent of AR-42's effects on reducing tumor load.

Aspects described herein disclose the effects of oral AR-42 in attenuating cachexia-induced weight loss, skeletal muscle atrophy, and prolonging survival in the Lewis Lung Carcinoma (LLC) tumor model of cancer cachexia. See, e.g., Expert Opin Drug Discov. Nov 1, 2009; 4(11): 1145-1155.

In another aspect, methods described herein utilize AR-42, also known as (S)-N-hydroxy-4-(3-methyl-2-phenylbutanamido)benzamide having the following chemical structure:

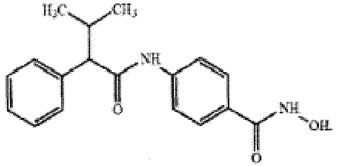

In yet another aspect, AR-42 includes salts, solvates, hydrates, anhydrous, co-crystalline and other crystalline forms and combinations. AR-42 can be formulated into a variety of dosage forms having increased stability, increased bioavailability, sustained release, and other properties.

In the colon 26 (C-26) tumor model of cancer cachexia, a fragment of the C26 tumor is grafted in isogenic BALB/c mice and the mice develop an undifferentiated carcinoma. Skeletal muscle atrophy (measured by muscle force and resistance to fatigue) correlated with the observed biochemical changes and the model was described as a "well standardized experimental model for research on cancer cachexia." BMC Cancer. 2010 Jul 8;10:363.

In one aspect, methods of suppressing cachexia in a mammal with cancer by administering the HDAC class 1 and 2b inhibitor AR-42 to the mammal in an amount effective to substantially maintain the mammal's weight compared to a mammal that does not receive the HDAC class 1 and 2b inhibitor AR-42 are provided.

In yet another aspect, the mammal's weight is not reduced by more than about 6% after about the first 15 days following treatment with AR-42.

In another aspect, the cancer is selected from the group consisting of pancreatic, colon, head, neck, gastric, and esophageal. In another aspect, the mammal is a human.

AR-42 can be administered in an amount of about 1 mg/kg to about 100 mg/kg of the mammal and administered at least once a day. In another aspect, AR-42 is administered twice a day in an amount of about 50 mg/kg of the mammal's weight.

In yet another aspect, levels of IL-6 are reduced by about 56% compared to a mammal that does not receive AR-42. In another aspect, levels of leukemia inhibitory factor (LIF) are reduced by about 88% compared to a mammal that does not receive AR-42. In another aspect, expression of Atrogin-1 mRNA is restored to basal levels compared to a mammal that does not receive AR-42.

In one aspect, expression of MuRF1 mRNA is restored to basal levels compared to a mammal that does not receive AR-42. In another aspect, cachexia-induced increase in IL-6Rα mRNA levels is reduced by about 85% compared to a mammal that does not receive AR-42.

In yet another aspect, cachexia-induced loss of adipose tissue is substantially restored compared to a mammal that does not receive AR-42. In another aspect, cachexia-induced reduction in skeletal muscle fiber size is restored by AR-42 compared to a mammal that does not receive AR-42.

Methods of maintaining skeletal muscle weight in a mammal having cancer comprising administering the HDAC class 1 and 2b inhibitor AR-42 to said mammal in an amount effective to maintain at least about 90% of said mammal's skeletal muscle weight over a period of time of at least fifteen days compared to a mammal that does not receive the HDAC class 1 and 2b inhibitor AR-42 are also disclosed.

In another aspect, methods of prolonging survival of a mammal having cancer comprising administering the HDAC class 1 and 2b inhibitor AR-42 to the mammal in an amount effective to substantially prolong survival of the mammal compared to a mammal that does not receive the HDAC class 1 and 2b inhibitor AR-42are disclosed. In yet another aspect, the mammal survives at least about 21 days after the administering of AR-42 to the mammal.

In another aspect, administration of AR-42 attenuated cachexia-induced weight loss and skeletal muscle atrophy and prolonged survival in mammals. Without being bound by theory, it is thought that the anti-cachexia effect is associated with the ability of AR-42 to reprogram cell metabolism and to downregulate IL-6 levels in diseased muscle tissues to suppress muscle wasting and other cachexia-related effects independent of AR-42's effects on reducing tumor load.

In one aspect, the anti-cachexia effects of AR-42 were measured by variety of techniques including qRT-PCT analysis of the expression of established mediators of muscle atrophy in cancer (e.g., Cancer Cell. 2008 Nov 4;14(5):369-81), measurement of the levels of anti-inflammatory cytokines in serum and gastrocnemius muscle tissues (Am J Pathol. 2011 Mar;178(3):1059-68), metabolomic profiling analysis (J Cachexia Sarcopenia Muscle. 2013 Jun;4(2):145-55), measuring the levels of free amino acids in muscle tissue (J Cachexia Sarcopenia Muscle. 2013 Jun;4(2):145-55; Am J Physiol Endocrinol Metab. 2007 Feb;292(2):E501-12), measuring the "glycolytic signature" of cachectic muscle by measuring levels of biochemical associated with the glycolysis pathway (Cachexia Sarcopenia Muscle. 2013 Jun;4(2):145-55), measuring the level of glycogen storage in cachectic muscle tissue (Cell Death Differ. 2012 Oct;19(10):1698-708), analyzing branched-chain amino acid metabolism in cachectic muscle (Int J Biochem Cell Biol. 2013 Oct;45(10):2163-72), and measuring levels of 2-hydroxybutyrate and ophthalmate in cachectic muscle (PLoS One. 2010 May 28;5(5):e10883; Int J Cancer. 2010 Feb 1;126(3):756-63).

The HDAC inhibitor AR-42, as described herein, can be administered to patient in need of treatment (e.g., a patient having cancer and exhibiting symptoms of cachexia). In one aspect, certain cancers are particularly associated with cachexia including, but not limited to pancreatic, gastric, head, neck, and esophageal ("cachexia-associated cancers"). In another aspect, the HDAC inhibitor AR-42 is administered to a patient in need of treatment.

As used herein, the term "substantially" refers to "most of," a "majority of," or at least 50%, 60%, 70%, 80%, and 90% of the weight or amount of, for example, of a mammal that does not have cancer.

The terms "treat," "reduce," "suppress," "inhibit," "prevent," or similar terms, as used herein, do not necessarily mean 100% or complete treatment or prevention. Rather, these terms refer to various degrees of treatment or prevention of a particular disease (e.g., 100%, 90%, 80%, 70%, 60%, 50%, 40%, 30%, 20%, 10%, 5%, or 1%) as recognized in the art as being beneficial.

The terms "treatment" or "prevention" also refer to delaying onset of a disease for a period of time or delaying onset indefinitely. The term "treatment" or "treating" refers to administering a drug or treatment to a patient or prescribing a drug to a patient (e.g., by a doctor, nurse, or other medical professional) where the patient or a third party (e.g., caretaker, family member, or health care professional) administers the drug or treatment. The term "amount effective" refers to an amount of a drug or treatment (e.g., the HDAC class I and IIb inhibitor AR-42) that will treat, reduce, suppress, inhibit, prevent disease(s) or condition(s) (e.g., cachexia) or prolong survival of a mammal with a disease or condition.

The term "prolong" or "prolonging" as used herein, refers to increasing time of survival of a mammal receiving treatment compared to a mammal that does not receive treatment. In this aspect, "prolonged survival" can refer to increasing the lifespan of the mammal by, for example, 1%, 5%, 10%, 20%, 30%, 40%, 50%, 60%, 70%, 80% or 90% of the lifespan of mammal that does not have cancer.

The HDAC inhibitor AR-42described herein can be administered orally, parenterally (IV, IM, depot-IM, SQ, and depot-SQ), sublingually, intranasally (inhalation), intrathecally, topically, or rectally. Dosage forms known to those of skill in the art are suitable for delivery of the HDAC inhibitorAR-42 described herein.

In one aspect, the HDAC inhibitor AR-42 is administered in an oral dosage form (e.g., pill, capsule, caplet, or tablet, etc.) to a patient diagnosed with a cancer associated with cachexia (e.g., pancreatic, bladder, gastric, head and neck).

The HDAC inhibitor AR-42 can be formulated into suitable pharmaceutical preparations such as tablets, capsules, or elixirs for oral administration or in sterile solutions or suspensions for parenteral administration. The HDAC inhibitor AR-42described herein can be formulated into pharmaceutical compositions using techniques and procedures well known in the art.

In one aspect, about 0.1 to 1000 mg, about 5 to about 100 mg, or about 10 to about 50 mg of the HDAC inhibitor AR-42, or a physiologically acceptable salt or ester can be compounded with a physiologically acceptable vehicle, carrier, excipient, binder, preservative, stabilizer, flavor, etc., in a unit dosage form as called for by accepted pharmaceutical practice. The amount of active substance in compositions or preparations comprising the HDAC inhibitor AR-42 is such that a suitable dosage in the range indicated is obtained.

In another aspect, the compositions can be formulated in a unit dosage form, each dosage containing from about 1 to about 500 mg, or about 10 to about 100 mg of the active ingredient. The term "unit dosage from" refers to physically discrete units suitable as unitary dosages for human subjects and other mammals, each unit containing a predetermined quantity of active material calculated to produce the desired therapeutic effect, in association with a suitable pharmaceutical excipient.

In one aspect, the HDAC inhibitor AR-42 is mixed with a suitable pharmaceutically acceptable carrier to form compositions. Upon mixing or addition of the compound(s), the resulting mixture may be a solution, suspension, emulsion, or the like. Liposomal suspensions may also be used as pharmaceutically acceptable carriers. These may be prepared according to methods known to those skilled in the art. The form of the resulting mixture depends upon a number of factors, including the intended mode of administration and the solubility of the compound in the selected carrier or vehicle. In one aspect, the effective concentration is sufficient for lessening or ameliorating at least one symptom of the disease, disorder, or condition treated and may be empirically determined.

In yet another aspect, AR-42 suppresses muscle wasting in cachexia as shown in the C-26 and LLC tumor models of cachexia. The pro-inflammatory cytokines, IL-6 and TNF, represent major pro-cachectic factors in the two models (31, 32). Cytokine profile analysis indicated that, while AR-42 had no effect on serum TNF levels in C-26 tumor-bearing mice, it reduced levels of serum IL-6 and intramuscular IL-6Rα mRNA expression. Nonetheless, these AR-42-treated C-26 tumor-bearing mice still exhibited elevated serum IL-6 levels and IL-6Rα mRNA compared to tumor-free mice, suggesting that decreased IL-6 signaling is not solely responsible for AR-42-mediated suppression of muscle wasting.

Mechanistically, the anti-cachectic effect of AR-42 is unique as the HDAC inhibitors valproic acid and trichostatin-A could not reverse muscle loss in C-26 tumor-bearing mice despite their ability to modulate the myostatin/follistatin axis (33). Similarly, our findings show that, unlike AR-42, vorinostat and romidepsin were ineffective in attenuating cachexia-induced weight loss in the C-26 model. This discrepancy was attributable to the greater ability of AR-42 to suppress the mRNA expression of the E3 ligases Atrogin-1 and MuRF1 in the muscles of tumor-bearing mice, which may reflect differences in their respective abilities to modulate global gene expression in skeletal muscles. Recent evidence suggests a mechanistic link between aberrant acetylation/expression of transcription factors and muscle wasting in diseased muscles, leading to dysregulated expression of cachexia-associated genes [review: (34)]. It was reported that the histone acetyltransferase activity of p300/CBP differentially regulates transcriptional activity and nuclear localization of Foxo family transcription factors in skeletal muscles (35), and that class I HDACs, especially HDAC1, play a crucial role in mediating nutrient deprivation- or muscle disuse-induced muscle atrophy by regulating expression of Foxo and its targets Atrogin-1 and MuRF1 (22).

RNA-seq analysis revealed the ability of AR-42 to reverse tumor-induced shift in gene expression. A total of 677 genes were identified that were differentially expressed by 4-fold or greater between AR-42- and vehicle-treated tumor-bearing mice. Conceivably, this large number of differentially expressed genes might arise from the effect of AR-42 on the transcriptional activity and/or expression of multiple transcription factors/regulators. In addition to Foxo1, AR-42 also modulated the expression of many other transcription factors/regulators, including C/EBPδ, Fos, Jun-b, DAXX, ERN1, HIF3α, MAFF, MAFK, and Mef2c (Figure 11). Among these transcription factors, the importance of Mef2c in the development of skeletal, cardiac, and smooth muscle is well documented (36), and the AP-1 signaling cascade has been implicated in cancer-associated muscle wasting (37).

It has been proposed that cachectic muscles in C-26 tumor-bearing mice exhibit tumor Warburg physiology, characterized by a high rate of glycolysis (38). Our metabolomic data reveals a pronounced reprogramming of skeletal muscle metabolism in C-26 tumor-bearing mice, which was completely reversed by AR-42. Moreover, the suppressive effect of AR-42 on the production of 2-hydroxybutyrate and opthalmate, biomarkers for insulin resistance (17) and oxidative stress (18), is noteworthy, as substantial evidence has associated insulin resistance (39, 40) and oxidative stress (41) with cachexia.

Mechanistically, the ability of AR-42 to maintain the integrity of skeletal muscles in tumor-bearing mice arises from its diverse, cumulative effects on tumor-induced changes in multiple transcriptional programs and metabolic phenotype. It is of therapeutic significance that oral administration of AR-42 at a late stage of tumor growth was still effective in slowing down the progression of muscle wasting in C-26 tumor-bearing mice. Together, these findings show that HDAC inhibitors (e.g., AR-42) can be used as part of a comprehensive therapeutic strategy for cancer cachexia, as described herein.

Pharmaceutical carriers or vehicles suitable for administration of the HDAC inhibitor AR-42 described herein include any such carriers suitable for the particular mode of administration. In addition, the active materials can also be mixed with other active materials that do not impair the desired action, or with materials that supplement the desired action, or have another action. The compounds may be formulated as the sole pharmaceutically active ingredient in the composition or may be combined with other active ingredients.

In another aspect, if the HDAC inhibitor AR-42 exhibits insufficient solubility, methods for solubilizing may be used. Such methods are known and include, but are not limited to, using co-solvents such as dimethylsulfoxide (DMSO), using surfactants such as TWEEN, and dissolution in aqueous sodium bicarbonate. Derivatives of the compounds, such as salts or prodrugs, may also be used in formulating effective pharmaceutical compositions.

The concentration of the compound is effective for delivery of an amount upon administration that lessens or ameliorates at least one symptom of the disorder for which the compound is administered. Typically, the compositions are formulated for single dosage administration.

In another aspect, the HDAC inhibitor AR-42 described herein may be prepared with carriers that protect them against rapid elimination from the body, such as time-release formulations or coatings. Such carriers include controlled release formulations, such as, but not limited to, microencapsulated delivery systems. The active compound can be included in the pharmaceutically acceptable carrier in an amount sufficient to exert a therapeutically useful effect in the absence of undesirable side effects on the patient treated. The therapeutically effective concentration may be determined empirically by testing the compounds in known in vitro and in vivo model systems for the treated disorder.

In another aspect, the HDAC inhibitor AR-42 and compositions described herein can be enclosed in multiple or single dose containers. The enclosed compounds and compositions can be provided in kits, for example, including component parts that can be assembled for use. For example, AR-42 in lyophilized form and a suitable diluent may be provided as separated components for combination prior to use. A kit may include AR-42 and a second therapeutic agent for co-administration. AR-42 and a second therapeutic agent may be provided as separate component parts. A kit may include a plurality of containers, each container holding one or more unit dose of the compounds described herein. In one aspect, the containers can be adapted for the desired mode of administration, including, but not limited to tablets, gel capsules, sustained-release capsules, and the like for oral administration; depot products, pre-filled syringes, ampoules, vials, and the like for parenteral administration; and patches, medipads, creams, and the like for topical administration.

The concentration of the exemplary HDAC inhibitor in the pharmaceutical composition will depend on absorption, inactivation, and excretion rates of the active compound, the dosage schedule, and amount administered as well as other factors known to those of skill in the art.

In another aspect, the active ingredient may be administered at once, or may be divided into a number of smaller doses to be administered at intervals of time. It is understood that the precise dosage and duration of treatment is a function of the disease being treated and may be determined empirically using known testing protocols or by extrapolation from in vivo or in vitro test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

If oral administration is desired, the compound can be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

Oral compositions will generally include an inert diluent or an edible carrier and may be compressed into tablets or enclosed in gelatin capsules. For the purpose of oral therapeutic administration, the active compound or compounds can be incorporated with excipients and used in the form of tablets, capsules, or troches. Pharmaceutically compatible binding agents and adjuvant materials can be included as part of the composition.

The tablets, pills, capsules, troches, and the like can contain any of the following ingredients or compounds of a similar nature: a binder such as, but not limited to, gum tragacanth, acacia, corn starch, or gelatin; an excipient such as microcrystalline cellulose, starch, or lactose; a disintegrating agent such as, but not limited to, alginic acid and corn starch; a lubricant such as, but not limited to, magnesium stearate; a glidant, such as, but not limited to, colloidal silicon dioxide; a sweetening agent such as sucrose or saccharin; and a flavoring agent such as peppermint, methyl salicylate, or fruit flavoring.

When the dosage unit form is a capsule, it can contain, in addition to material of the above type, a liquid carrier such as fatty oil. In addition, dosage unit forms can contain various other materials, which modify the physical form of the dosage unit, for example, coatings of sugar and other enteric agents. The compounds can also be administered as a component of an elixir, suspension, syrup, wafer, chewing gum or the like. A syrup may contain, in addition to the active compounds, sucrose as a sweetening agent and certain preservatives, dyes and colorings, and flavors.

The active materials can also be mixed with other active materials that do not impair the desired action, or with materials that supplement the desired action. The HDAC inhibitor AR-42 can be used, for example, in combination with an antitumor agent, a hormone, a steroid, or a retinoid. The antitumor agent may be one of numerous chemotherapy agents such as an alkylating agent, an antimetabolite, a hormonal agent, an antibiotic, colchicine, a vinca alkaloid, L-asparaginase, procarbazine, hydroxyurea, mitotane, nitrosoureas or an imidazole carboxamide. Suitable agents include those agents which promote depolarization of tubulin. Examples include colchicine and vinca alkaloids, including vinblastine and vincristine.

In another aspect, the HDAC inhibitor AR-42 described herein can be co-administered or administered before or after immunization of a patient with a vaccine to enhance the immune response to the vaccine. In one aspect the vaccine is a DNA vaccine, for example, and HPV vaccine.

In one aspect, solutions or suspensions used for parenteral, intradermal, subcutaneous, or topical application can include any of the following components: a sterile diluent such as water for injection, saline solution, fixed oil, a naturally occurring vegetable oil such as sesame oil, coconut oil, peanut oil, cottonseed oil, and the like, or a synthetic fatty vehicle such as ethyl oleate, and the like, polyethylene glycol, glycerin, propylene glycol, or other synthetic solvent; antimicrobial agents such as benzyl alcohol and methyl parabens; antioxidants such as ascorbic acid and sodium bisulfite; chelating agents such as ethylenediaminetetraacetic acid (EDTA); buffers such as acetates, citrates, and phosphates; and agents for the adjustment of tonicity such as sodium chloride and dextrose. Parenteral preparations can be enclosed in ampoules, disposable syringes, or multiple dose vials made of glass, plastic, or other suitable material. Buffers, preservatives, antioxidants, and the like can be incorporated as required.

Where administered intravenously, suitable carriers include, but are not limited to, physiological saline, phosphate buffered saline (PBS), and solutions containing thickening and solubilizing agents such as glucose, polyethylene glycol, polypropyleneglycol, and mixtures thereof. Liposomal suspensions including tissue-targeted liposomes may also be suitable as pharmaceutically acceptable carriers. These may be prepared according to methods known in the art.

In another aspect, the HDAC inhibitor AR-42 may be prepared with carriers that protect the compound against rapid elimination from the body, such as time-release formulations or coatings. Such carriers include controlled release formulations, such as, but not limited to, implants and microencapsulated delivery systems, and biodegradable, biocompatible polymers such as collagen, ethylene vinyl acetate, polyanhydrides, polyglycolic acid, polyorthoesters, polylactic acid, and the like. Methods for preparation of such formulations are known to those skilled in the art.

In yet another aspect, compounds employed in the methods of the disclosure may be administered enterally or parenterally. When administered orally, compounds employed in the methods of the disclosure can be administered in usual dosage forms for oral administration as is well known to those skilled in the art. These dosage forms include the usual solid unit dosage forms of tablets and capsules as well as liquid dosage forms such as solutions, suspensions, and elixirs. When the solid dosage forms are used, they can be of the sustained release type so that the compounds employed in the methods described herein need to be administered only once or twice daily.

The oral dosage forms can be administered to the patient 1, 2, 3, or 4 times daily. The HDAC inhibitor AR-42 described herein can be administered either three or fewer times, or even once or twice daily. Hence, the HDAC inhibitor compound AR-42 employed in the methods of the disclosure be administered in oral dosage form. Whatever oral dosage form is used, they can be designed so as to protect the compounds employed in the methods described herein from the acidic environment of the stomach. Enteric coated tablets are well known to those skilled in the art. In addition, capsules filled with small spheres each coated to protect from the acidic stomach, are also well known to those skilled in the art.

The terms "therapeutically effective amount" and "therapeutically effective period of time" are used to denote treatments at dosages and for periods of time effective to reduce neoplastic cell growth. As noted above, such administration can be parenteral, oral, sublingual, transdermal, topical, intranasal, or intrarectal. In one aspect, when administered systemically, the therapeutic composition can be administered at a sufficient dosage to attain a blood level of the compounds of from about 0.1 µM to about 100 mM. For localized administration, much lower concentrations than this can be effective, and much higher concentrations may be tolerated. One of skill in the art will appreciate that such therapeutic effect resulting in a lower effective concentration of an HDAC inhibitor or AR-42 may vary considerably depending on the tissue, organ, or the particular animal or patient to be treated. It is also understood that while a patient may be started at one dose, that dose may be varied overtime as the patient's condition changes.

It should be apparent to one skilled in the art that the exact dosage and frequency of administration will depend on the particular compounds employed in the methods of the disclosure administered, the particular condition being treated, the severity of the condition being treated, the age, weight, general physical condition of the particular patient, and other medication the individual may be taking as is well known to administering physicians who are skilled in this art.

### EXAMPLES

The following examples illustrate aspects described herein.

### Example 1

### Cancer cachexia models

### C-26 model

In one aspect, tumors were established by subcutaneous injection of C-26 cells (0.5 x 106 cells in 0.1 ml) into the right flank of male CD2F1 mice (approximately 6 weeks of age; Harlan Laboratories, Indianapolis, IN)(11). Tumor-bearing mice, as well as tumor-free mice serving as non-cachectic controls, were randomized into groups that were treated with either AR-42 (50 mg/kg, p.o. by gavage, every other day; Arno Therapeutics, Inc., Flemington, NJ) or vehicle (0.5% methylcellulose (w/v) and 0.1% Tween-80 (v/v) in sterile water) starting 6 days after cell injection. To investigate the effect of delayed treatment, treatments with drug and/or vehicle were started 6, 10 and 12 days after cancer cell injection.

In another aspect, the effects of AR-42 were compared to other HDAC inhibitors. In this aspect, additional groups of C-26 tumor-bearing mice were treated with vorinostat (50 mg/kg, p.o., once daily) and romidepsin (0.6 mg/kg; i.p., twice weekly) (ChemieTek (Indianapolis, IN)).

### LLC model

In another aspect, subcutaneous tumors were established in male C57BL/6 mice (approximately 6 weeks of age; Harlan) by injection of 0.5 x 106 LLC cells into the right flank. Treatment with AR-42 and vehicle was performed as for the C-26 model beginning 6 days after cell injection. In both models, body weights and food consumption were monitored daily and tumor size was measured no less than every two days. Mice were fasted for 2 hours prior to sacrifice, at which time, hind limb muscles, heart, spleen, epididymal fat, and blood were collected and the weights of the solid tissues were measured. Muscle samples were frozen in liquid nitrogen-chilled 2-methylbutane and then stored at -80°C until analysis.

### Example 2

### Grip strength measurement

Forelimb grip strength was measured in mice using a Digital Grip Strength Meter (Columbus Instruments, Columbus, OH). Five measurements were taken from each mouse, the average of which was designated as the mouse's grip strength.

### Morphometric analysis of muscle fiber size

Ten-µm sections were cut from frozen skeletal muscle samples using a cryostat (Leica) and then stained with H&E. Images were captured using an Olympus BX51 microscope (Olympus America, Inc.) and muscle fiber cross-sectional areas were determined using Olympus CellSens 1.11 software. Measurements were obtained from five different sections of muscle from each of five mice from each group.

### RNA isolation, qRT-PCR, and RNA-seq analysis

Total RNA was isolated from homogenized gastrocnemius muscles (n = 3/group) with TRIzol reagent (Life Technologies, Carlsbad, CA) and then purified using the RNAeasy Mini Kit (Qiagen, Valencia, CA). qRT-PCR was performed as described previously (42) by using the Bio-Rad CFX96 Real-Time PCR Detection System with iQ SYBR Green Supermix (Bio-Rad, Hercules, CA). Primer sequences are listed in Fig. 8. RNA-seq library generation and data analysis were performed at The Ohio State University Comprehensive Cancer Center (OSUCCC) Nucleic Acid Shared Resource.

### Metabolomic and cytokine profiling

Gastrocnemius muscles and sera were collected at day 17 post-cell injection from each treatment group (n = 8/group). Muscle was submitted to Metabolon, Inc. (Durham, NC) for metabolomic analysis of 270 metabolic intermediates via proprietary mass spectrometry platforms. Serum was submitted to Eve Technologies (Alberta, Canada) for analysis of 32 cytokines using a mouse cytokine array (32-plex panel).

### Statistical analysis

Data analysis was conducted by using SAS 9.3 software (SAS, Inc; Cary, NC). For the experiments with repeated measures, data were analyzed by mixed effect models, incorporating observational dependencies across each subject. For other experiments involving independent groups, data were analyzed by ANOVA. For the time-to-event experiment (Fig. 2B), the difference in survival functions were compared by log-rank tests. Multiplicities were adjusted by Holm's method to control the overall family-wise error rate at 0.05. RNA-seq data were analyzed by using Ingenuity Pathway Analysis (IPA) software (Ingenuity Systems, Redwood City, CA). Only genes with >4-fold change and P<0.05 were selected for pathway analysis.

### Example 3

### AR-42 suppresses cancer cachexia in the C-26 colon adenocarcinoma model

In one aspect, mice were treated orally by gavage with AR-42 (50mg/kg) or vehicle every other day starting 6 days after injection C-26 cells, when palpable tumors had formed. While the vehicle group showed a large drop in body weight starting at day 12, AR-42-treated mice maintained their weight at levels comparable to that of tumor-free controls (Fig. 1A, left). By the study endpoint (day 15), the magnitude of the weight loss, after deducting the mass of the tumors (1cm³ volume = 1gram mass), reached >20% for the vehicle-treated group, and 6% for AR-42-treated mice (center). This effect cannot be attributed to decreased tumor burden, since AR-42 did not alter tumor growth relative to vehicle (right), or to increased food intake, since average daily consumption was comparable in the AR-42-treated and vehicle-treated groups, and less than that in tumor-free mice (Fig. 1C). The AR-42-treated mice, despite their large tumor burden, were alert, responsive, active, and lacked the hunched posture and rough haircoat observed in vehicle-treated counterparts by the end point of this study (Fig. 1B).

### Example 4

### AR-42 protects muscle against cachexia-induced atrophy

Consistent with the preservation of body weight, skeletal muscle mass was preserved in AR-42-treated tumor-bearing mice. Indicative of cachexia, the weights of gastrocnemius, tibialis anterior and quadriceps from vehicle-treated tumor-bearing (tumor-bearing/vehicle) mice were reduced by 20.6%, 10.5%, and 18.1%, respectively, relative to corresponding muscles from tumor-free control mice, while those in AR-42-treated tumor-bearing (tumor-bearing/AR-42) mice were reduced by 9.6%, 0.8%, and 5.8%, respectively (Fig. 1D).

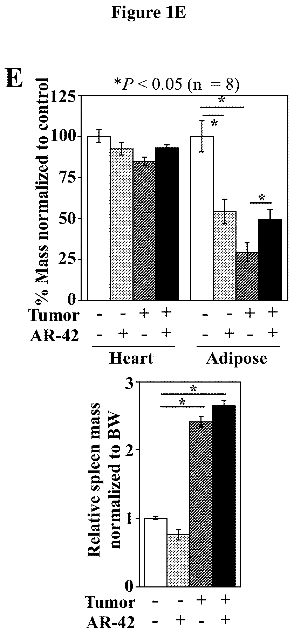

Tumor-bearing/vehicle mice exhibited other hallmarks of cachexia, including significant losses of cardiac and, particularly, adipose tissue mass (29.3±6.0% of tumor-free control), which were ameliorated by AR-42 treatment (Fig. 1E, upper). Interestingly, AR-42 significantly reduced the mass of adipose tissue by approximately 50% in tumor-free mice yet restored the loss of adipose tissue mass in tumor-bearing mice to a level comparable to that of tumor-free/AR-42 mice, a dichotomous effect suggesting its ability to maintain lipid homeostasis.

In another aspect, C-26 tumor-bearing mice exhibited grossly enlarged spleens relative to tumor-free control mice (11), which were not improved by AR-42 (Fig. 1E, lower). As splenomegaly in C-26 tumor-bearing mice results from expansion of myeloid-derived suppressor cells and other immune cells in the spleen (12), this finding suggests AR-42 was acting predominantly on the muscle rather than through an immunologic mechanism.

The protective effect of AR-42 against muscle wasting was manifested by the abrogation of cachexia-induced reduction in skeletal muscle fiber size. Tumor-bearing/vehicle mice exhibited a 48.2% decrease relative to the tumor-free control in mean cross-sectional area of muscle fibers at day 15 (1297.6±638.8 versus 2503.5±917.5 µm²), which was restored by AR-42 (2146.3±923.4 µm²)(Fig. 2A, left). The prominent shift in fiber size distribution to smaller cross-sectional area in cachectic muscles of tumor-bearing/vehicle mice was reversed by AR-42 (Fig. 2A, right)

### AR-42 prolongs the survival of C-26 tumor-bearing mice

The effects of AR-42 were compared to other HDAC inhibitors (i.e., vorinostat and romidepsin) in C-26 tumor-bearing mice. Starting on day 6 after tumor cell injection, mice were treated continuously with AR-42 (50 mg/kg every other day, orally), vorinostat [50 mg/kg once daily, orally (13)], romidepsin [0.6 mg/kg twice weekly, i.p. (14)], or vehicle, until weight loss, as determined by daily body weight measurements and subtraction of tumor mass, reached 20% of starting weight. As shown, oral AR-42 was effective in protecting these mice from tumor-associated wasting, with 100% cumulative survival at day 21 when tumor volume reached the threshold for euthanasia (Fig. 2B). In contrast, vorinostat and romidepsin showed limited or no appreciable protective effects on body weight. Moreover, tumor-bearing/AR-42 mice were alert, responsive, active, and appeared healthy at 21 days after tumor cell injection, in contrast to vehicle- (Day 15), romidepsin- (Day 16), and vorinostat-treated mice (Day 18)(Fig. 2C).

### Example 5

### Differential effects on the regulation of skeletal muscle protein turnover

Skeletal muscle mass is regulated by a balance between protein synthesis and degradation. Without being bound by theory, it is believed that the differential anti-cachectic effect of AR-42 versus vorinostat and romidepsin may be attributable to differences in their ability to regulate pathways governing protein turnover. This was supported by the suppressive effect of AR-42 on the mRNA expression of Atrogin-1/MAFbx and, MuRF1, two E3 ligases involved in ubiquitin-mediated skeletal muscle protein degradation (15, 16) (Fig. 2D).

qPCR analysis of gastrocnemius muscles revealed a significant increase in Atrogin-1 and MuRF1 mRNA levels (29.4±3.5-fold and 25.8±3.9-fold, respectively) in cachectic muscle (tumor-bearing/vehicle; n=8) relative to the tumor-free/vehicle control (n=6). AR-42 was able to restore expression of Atrogin-1 (2.7±0.7-fold) and MuRF1 (1.1 ± 0.2-fold) mRNA to basal levels (n=8). Vorinostat (n=8) and romidepsin (n=5) also significantly reduced the mRNA expression of these two E3 ligases in cachectic muscles, but to a lesser extent than AR-42 (Atrogin-1/MuRF1: vorinostat, 9.6±1.8/5.5±1.1-fold; romidepsin, 19.6±3.1/14.6±3.3-fold)(Fig. 2D).

To confirm that the anti-cachectic activity of AR-42 was not specific to the C-26 model, it was also evaluated in the LLC model. C57BL/6 mice bearing subcutaneous LLC tumors were treated with AR-42 (50 mg/kg, p.o., every other day) starting on day 6 after tumor cell injection, and continuing until day 20 when hind limb muscles were harvested at sacrifice. As shown in Fig. 7, AR-42 protected LLC tumor-bearing C57B1/6 mice from loss of muscle mass (gastrocnemius: vehicle, 81.7±3.7% of non-cachectic control; AR-42, 92.2±3.5%; tibialis anterior: vehicle, 80.3±4.0%; AR-42, 93.4±3.9%; quadriceps: vehicle, 84.4±4.6%; AR-42, 93.4±4.8%; all *P* values <0.05, n=8).

### Example 6

### AR-42 maintains metabolic integrity of muscle in tumor-bearing mice

With cachexia, skeletal muscles undergo complex metabolic changes in response to tumor/host-derived inflammatory and neuroendocrine stressors (1). Accordingly, we conducted metabolic profiling analysis to investigate the effect of AR-42 on cachexia-induced shifts in metabolic phenotype in skeletal muscle. Tumor-free and C-26 tumor-bearing mice were treated with vehicle or AR-42 as aforementioned, and gastrocnemius muscles were collected at day 17 for metabolomic analysis. Comparison of muscle biochemical profiles among the four groups (n=8/group) revealed the ability of AR-42 to restore cachexia-induced metabolic changes in skeletal muscles, which are summarized as follows.

**Glycolysis.** Cachectic muscles from tumor-bearing/vehicle mice showed significantly lower levels of glucose and key glycolytic intermediates than tumor-free controls (Fig. 3A). AR-42 reversed these metabolic changes, restoring the intramuscular levels of glucose and intermediates to or, in some cases, above baseline levels detected in tumor-free/vehicle mice. Moreover, elevated glucose was shunted into sorbitol-fructose biosynthesis and pentose phosphate pathways, leading to increased production of sorbitol, fructose, and ribose, a metabolite derived from the pentose phosphate pathway.

**Glycogen stores.** Muscle from tumor-bearing/vehicle mice showed significant decreases in short-chain malto-oligosaccharides and glucose 1-phosphate (Fig. 3B), suggesting the depletion of glycogen stores in cachectic muscles. AR-42 treatment significantly replenished these glycogen metabolic intermediates.

**Free amino acids.** Consistent with the increased protein degradation that characterizes muscle wasting, a large number of free amino acids were significantly elevated in cachectic muscles from tumor-bearing/vehicle mice relative to that in tumor-free/vehicle mice (Fig. 4), indicative of a cachectic phenotype. Similarly, several amino acid derivatives/metabolites that function as neurotransmitters, including kynurenin, *N*-acetyl-aspartyl-glutamate, and γ-aminobutyrate, were elevated. In contrast, alanine, which is released from muscles to support liver gluconeogenesis, was reduced in cachectic muscles. This cachectic phenotype was reversed by AR-42 treatment, indicating its ability to block muscle protein degradation.

**Organic acids.** The amino acid metabolites 2-hydroxybutyrate and ophthalmate are biomarkers for insulin resistance (17) and oxidative stress (18), respectively. The increase in these two organic acids in muscles of tumor-bearing/vehicle mice (Fig. 4) suggests that cachexia promotes insulin resistance and oxidative stress, which, in turn, exacerbates muscle wasting. AR-42 dramatically reduced the two biomarkers to levels comparable to those measured in tumor-free mice.

### Example 7

### AR-42 suppresses cancer cachexia by targeting multiple pro-cachexia drivers

To shed light onto the mechanism by which AR-42 mediated its anti-cachectic effect, sera and gastrocnemius muscle from vehicle- or AR-42-treated tumor-free and C-26 tumor-bearing mice were used for cytokine profiling analysis and whole transcriptome shotgun sequencing (RNA-seq), respectively.

Cytokine profiles. Of 32 cytokines examined (Figure 10), IL-6 and leukemia inhibitory factor (LIF), two well-recognized cachexia drivers (19), were significantly increased in the sera of tumor-bearing/vehicle mice relative to that of tumor-free/vehicle mice (IL-6; 230±105 versus 2.9±1.3 pg/ml; LIF, 19.7±9.3 versus 1.7±1.5 pg/ml) (Fig. 5A, upper), while no significant differences were noted with other cytokines. AR-42 reduced IL-6 and LIF levels by 56% and 88%, respectively, in tumor-bearing mice (IL-6, 102±38 pg/ml; LIF, 3.8±1.6 pg/ml) compared to the vehicle-treated counterparts. In light of the ability of AR-42 to blunt cachexia-associated increases in IL-6, we examined the effect of AR-42 on intramuscular mRNA levels of IL-6 receptor alpha chain (IL-6Rα). IL-6Rα mRNA was significantly elevated (13±1.4-fold) in muscle of tumor-bearing/vehicle mice (n=9) compared to that of tumor-free mice (n=6). AR-42 reduced this cachexia-induced increase by 85% (2.0±0.2-fold; n=10)(Fig. 5, lower). These findings suggest that AR-42 inhibits muscle wasting, in part, by blocking IL-6 signaling.

**RNA-seq analysis.** Principal component analysis of the RNA-seq data revealed a pronounced effect of the C-26 tumor on the global gene expression pattern in the muscle of tumor-bearing/vehicle mice relative to tumor-free/vehicle counterparts (Fig. 5B, left). While AR-42 had no appreciable effect on the pattern of gene expression in the non-cachectic muscle of tumor-free mice, it reversed the tumor-induced shift in gene expression in cachectic muscle to a state close to that in tumor-free mice. Pursuant to this, we conducted pairwise analysis of differentially expressed genes between tumor-bearing/vehicle mice and the other three treatment groups, the results of which are represented in a Venn diagram (Fig. 5B, right). The largely overlapping areas among paired analyses suggest that AR-42 restores, to a great extent, tumor-induced changes in global gene expression.

Pairwise comparison of gene expression in muscles from vehicle- and AR-42-treated tumor-bearing mice revealed a total of 677 genes with 4-fold or greater differential expression (376 upregulated and 301 downregulated) (Figure 10). Analysis of these genomic data for their functional and disease associations using Ingenuity Pathway Analysis (IPA) revealed that 66 of these differentially expressed genes were annotated to categories of atrophy, contractility, development, and muscle morphology, and skeletal muscle cell size, muscle cell death, and protein catabolism (Figure 11).

Of these muscle function- and disease-associated genes, the effects of AR-42 on the following six genes are noteworthy in light of their demonstrated links with cancer-induced cachexia. These include *Foxo1* (encoding Forkhead box protein O1) (20-23) and its target genes *Trim63* (MuRF1) and *Fbxo32* (Atrogin-1) (24, 25), *PNPLA2* [adipose triglyceride lipase (ATGL)] (26, 27), *UCP3* (uncoupling protein 3) (28, 29), and *Mef2c* [myogenic transcription factor myocyte enhancer factor] (30) (Fig. 5C). Validation of the RNA-seq data for these six genes by qRT-PCR showed a high correlation between the data sets for the relative mRNA expression levels among the four treatment groups (Fig. 5D).

### Example 8

### Delayed treatment with AR-42 remains effective in suppressing muscle wasting

The above findings demonstrate the efficacy of oral AR-42 in suppressing cancer-associated muscle wasting by restoring metabolic and gene expression profiles in skeletal muscle. In those experiments, treatment was initiated early in the progression of cachexia when overt signs of wasting were undetectable. To investigate whether later initiation of AR-42 treatment remains protective against cachexia, C-26 tumor-bearing mice were treated with AR-42 (50 mg/kg, p.o., every other day) starting at 6, 10 and 12 days after tumor cell injection.

Consistent with our earlier data (Fig. 1), tumor-bearing/vehicle mice lost 19% of body weight (tumor excluded) by day 17. In contrast, treatment with AR-42 starting at day 6 (D6), 10 (D10), or 12 (D12) limited weight loss to 6%, 11%, and 12%, respectively (n=8)(Fig. 6A, left), without appreciable effects on tumor growth (right). Moreover, AR-42-treated mice exhibited signs better health than their vehicle-treated counterparts (Fig. 6B). This protective effect of AR-42 was reflected in the preservation of gastrocnemius weight and, to a lesser extent, that of tibialis anterior and quadriceps muscles (Fig. 6C). Consistent with the protective effect on muscle mass, handgrip dynamometry indicated that AR-42 helped preserve forelimb muscle strength in all drug-treated groups relative to the vehicle-treated control at day 15 and 16 (Fig. 6D).

### Example 9

Tumor-bearing/vehicle mice exhibited other hallmarks of cachexia, including significant losses of cardiac and, particularly, adipose tissue mass (29.3±6.0% of tumor free control), which were ameliorated by AR-42 treatment (Fig. IE, upper). Interestingly, AR-42 significantly reduced the mass of adipose tissue by approximately 50% in tumor free mice yet restored the loss of adipose tissue mass in tumor-bearing mice to a level comparable to that of tumor-free/AR-42 mice, a dichotomous effect suggesting its ability to maintain lipid homeostasis.

C-26 tumor-bearing mice exhibited grossly enlarged spleens relative to tumor-free control mice (11), which was not improved by AR-42 (Fig. 1E, lower). As splenomegaly in C-26 tumor-bearing mice results from expansion of myeloid-derived suppressor cells and other immune cells in the spleen (12), this finding suggests AR-42 was acting predominantly on the muscle rather than through an immunologic mechanism.

The protective effect of AR-42 against muscle wasting was manifested by the abrogation of cachexia-induced reduction in skeletal muscle fiber size. Tumor-bearing/vehicle mice exhibited a 48.2% decrease relative to the tumor-free control in mean cross-sectional area of muscle fibers at day 15 (1297.6±638.8 versus 2503.5±917.5 µm₂), which was restored by AR-42 (2146.3±923.4 µm₂)(Fig. 2A, left). The prominent shift in fiber size distribution to smaller cross-sectional area in cachectic muscles of tumor-bearing/vehicle mice was reversed by AR-42 (Fig. 2A, right).

### Example 10

### Differential effects on the regulation of skeletal muscle protein turnover

As skeletal muscle mass is regulated by a balance between protein synthesis and degradation, the differential anti-cachectic effect of AR-42 versus vorinostat and romidepsin may be attributable to differences in their ability to regulate pathways governing protein turnover. This was supported by the suppressive effect of AR-42 on the mRNA expression of Atrogin-1/MAFbx and, MuRF1, two E3 ligases involved in ubiquitin-mediated skeletal muscle protein degradation (15, 16) (Fig. 2D). As expected, qPCR analysis of gastrocnemius muscles revealed a significant increase in Atrogin-1 and MuRF1 mRNA levels (29.4±3.5-fold and 25.8±3.9-fold, respectively) in cachectic muscle (tumor-bearing/vehicle; n=8) relative to the tumor-free/vehicle control (n=6). AR-42 was able to restore expression of Atrogin-1 (2.7±0.7-fold) and MuRF1 (1.1 ± 0.2-fold) mRNA to basal levels (n=8). Vorinostat (n=8) and romidepsin (n=5) also significantly reduced the mRNA expression of these two E3 ligases in cachectic muscles, but to a lesser extent than AR-42 (Atrogin-1/MuRF1: vorinostat, 9.6±1.8/5.5±1.1-fold; romidepsin, 19.6±3.1/14.6±3.3-fold) (Fig. 2D).

### Example 11

### Cells

Cultured C-26 and LLC cells were maintained in fetal bovine serum (FBS)-supplemented (10%) RPMI 1640 medium and DMEM medium (Invitrogen, Carlsbad, CA), respectively, at 37°C in a humidified incubator with 5% CO₂. For injection into mice for cancer cachexia models, cells were harvested by trypsinization, pelleted in the FBS-supplemented culture medium, and then resuspended in sterile PBS at a concentration of 5 × 10⁶ cells/ml.

### Mice

CD2F1 and C57BL/6 mice were group-housed under conditions of constant photoperiod (12-hour light/12-hour dark), temperature and humidity with *ad libitum* access to water and standard diet. Mice were briefly anesthetized (isoflurane, 3-4%) during administration of drugs (AR-42, vorinostat, vehicle) by oral gavage. Food consumption was estimated by weighing food in each cage daily and dividing the daily decrease in food by the number of mice in the cage. Tumor volumes were calculated from caliper measurements using a standard formula (length x width² x π/6).

### Grip strength measurement

To measure forelimb grip strength, each mouse was held by the base of its tail and lowered over the apparatus until its forepaws grasped the pull bar. The mouse was then gently pulled horizontally in a straight line away from the grip meter until the mouse released the bar, and the maximum force attained was recorded. Five measurements were taken from each mouse, the average of which was designated as the mouse's grip strength.

### RNA-seq library generation and data analysis pipeline

RNA quality was assessed on an Agilent 2100 Bioanalyzer using a Pico RNA chip and the input total RNA amount was assessed using Agilent Qubit RNA assay. Transcriptome libraries were prepared using the Illumina TruSeq RNA Sample Preparation Kit V2. The resultant libraries were assessed for quantity and quality using Agilent Qubit DNA assay and with PerkinElmer Labchip DNA GX analysis, respectively. All libraries were mixed in equal proportions generating pools of samples that would yield approximately 40 million passed filter reads when sequenced on Illumina HiSeq 2500 sequencer. The raw sequencing data from the Illumina HiSeq CASAVA pipeline were assessed for quality using FastQC, RNASeQC and RSeQC software. Subsequent analyses were as follows: demultiplexed passed filter sequencing reads were aligned to GRCm38/mm10 using TopHat 2 (v2.0.7) RNAseq aligner; CuffLinks 2 (c2.1.1) was used for assembling the aligned reads to UCSC mm10 gene annotation; CuffCompare and CuffMerge was used to compile aligned reads to mm10 genes and merge assembled transcripts into a custom gene annotation; CuffDiff was used to compare differential gene expression associated with each treatment group.

### Example 12

To confirm that the anti-cachectic activity of AR-42 was not specific to the C-26 model, it was also evaluated in the LLC model. C57BL/6 mice bearing subcutaneous 11 LLC tumors were treated with AR-42 (50 mg/kg, p.o., every other day) starting on day 6 after tumor cell injection, and continuing until day 20 when hind limb muscles were harvested at sacrifice.

As shown in Figure 7, AR-42 protects against cancer-induced muscle wasting in the LLC mouse model of cachexia. Effects of AR-42 versus vehicle on the mass of hindlimb muscles, including gastrocnemius, tibialis anterior, and quadriceps, in both tumor-free and tumor-bearing mice were compared to that of vehicle-treated tumor-bearing mice. Mice were treated in the same manner as described in Fig. 1A, except that mice were sacrificed at day 20 after tumor cell injection. Data are presented as means ± S.D. (n = 8); (gastrocnemius: vehicle, 81.7±3.7% of non-cachectic control; AR-42, 92.2±3.5%; tibialis anterior: vehicle, 80.3±4.0%; AR-42, 93.4±3.9%; quadriceps: vehicle, 84.4±4.6%; AR-42, 93.4±4.8%; all P values <0.05, n=8).

### REFERENCES

1. Fearon KC, Glass DJ, Guttridge DC. Cancer cachexia: mediators, signaling, and metabolic pathways. Cell Metab 2012; 16: 153-66.
2. Tisdale MJ. Cachexia in cancer patients. Nat Rev Cancer 2002; 2: 862-71.
3. von Haehling S, Anker SD. Cachexia as a major underestimated and unmet medical need: facts and numbers. J Cachexia Sarcopenia Muscle 2010; 1: 1-5.
4. Tisdale MJ. Mechanisms of cancer cachexia. Physiol Rev 2009; 89: 381-410.
5. Lee SJ, Glass DJ. Treating cancer cachexia to treat cancer. Skelet Muscle 2011; 1: 2.
6. Maccio A, Madeddu C, Mantovani G. Current pharmacotherapy options for cancer anorexia and cachexia. Expert Opin Pharmacother 2012; 13: 2453-72.
7. Kulp SK, Chen CS, Wang DS, Chen CY, Chen CS. Antitumor effects of a novel phenylbutyrate-based histone deacetylase inhibitor, (S)-HDAC-42, in prostate cancer. Clin Cancer Res 2006; 12: 5199-206.
8. Lu YS, Kashida Y, Kulp SK, Wang YC, Wang D, Hung JH, et al. Efficacy of a novel histone deacetylase inhibitor in murine models of hepatocellular carcinoma. Hepatology 2007; 46: 1119-30.
9. Sargeant AM, Rengel RC, Kulp SK, Klein RD, Clinton SK, Wang YC, et al. OSU-HDAC42, a histone deacetylase inhibitor, blocks prostate tumor progression in the transgenic adenocarcinoma of the mouse prostate model. Cancer Res 2008; 68: 3999-4009.
10. Yang YT, Balch C, Kulp SK, Mand MR, Nephew KP, Chen CS. A rationally designed histone deacetylase inhibitor with distinct antitumor activity against ovarian cancer. Neoplasia 2009; 11: 552-63, 3 p following 63.
11. Acharyya S, Ladner KJ, Nelsen LL, Damrauer J, Reiser PJ, Swoap S, et al. Cancer cachexia is regulated by selective targeting of skeletal muscle gene products. J Clin Invest 2004; 114: 370-8.
12. Mundy-Bosse BL, Lesinski GB, Jaime-Ramirez AC, Benninger K, Khan M, Kuppusamy P, et al. Myeloid-derived suppressor cell inhibition of the IFN response in tumor-bearing mice. Cancer Res 2011; 71: 5101-10.
13. Lucas DM, Alinari L, West DA, Davis ME, Edwards RB, Johnson AJ, et al. The novel deacetylase inhibitor AR-42 demonstrates pre-clinical activity in B-cell malignancies in vitro and in vivo. PLoS One 2010; 5: e10941.
14. Sasakawa Y, Naoe Y, Inoue T, Sasakawa T, Matsuo M, Manda T, et al. Effects of FK228, a novel histone deacetylase inhibitor, on human lymphoma U-937 cells in vitro and in vivo. Biochem Pharmacol 2002; 64: 1079-90.
15. Bodine SC, Latres E, Baumhueter S, Lai VK, Nunez L, Clarke BA, et al. Identification of ubiquitin ligases required for skeletal muscle atrophy. Science 2001; 294: 1704-8.
16. Lecker SH, Jagoe RT, Gilbert A, Gomes M, Baracos V, Bailey J, et al. Multiple types of skeletal muscle atrophy involve a common program of changes in gene expression. FASEB J 2004; 18: 39-51.
17. Gall WE, Beebe K, Lawton KA, Adam KP, Mitchell MW, Nakhle PJ, et al. alpha-hydroxybutyrate is an early biomarker of insulin resistance and glucose intolerance in a nondiabetic population. PLoS One 2010; 5: e10883.
18. Soga T, Baran R, Suematsu M, Ueno Y, Ikeda S, Sakurakawa T, et al. Differential metabolomics reveals ophthalmic acid as an oxidative stress biomarker indicating hepatic glutathione consumption. J Biol Chem 2006; 281: 16768-76.
19. Tisdale MJ. Biology of cachexia. J Natl Cancer Inst 1997; 89: 1763-73.
20. Kamei Y, Miura S, Suzuki M, Kai Y, Mizukami J, Taniguchi T, et al. Skeletal muscle FOXO1 (FKHR) transgenic mice have less skeletal muscle mass, downregulated Type I (slow twitch/red muscle) fiber genes, and impaired glycemic control. J Biol Chem 2004; 279: 41114-23.
21. Reed SA, Sandesara PB, Senf SM, Judge AR. Inhibition of FoxO transcriptional activity prevents muscle fiber atrophy during cachexia and induces hypertrophy. FASEB J 2012; 26: 987-1000.
22. Beharry AW, Sandesara PB, Roberts BM, Ferreira LF, Senf SM, Judge AR. HDAC1 activates FoxO and is both sufficient and required for skeletal muscle atrophy. J Cell Sci 2014; 127: 1441-53.
23. Sandri M, Sandri C, Gilbert A, Skurk C, Calabria E, Picard A, et al. Foxo transcription factors induce the atrophy-related ubiquitin ligase atrogin-1 and cause skeletal muscle atrophy. Cell 2004; 117: 399-412.
24. Gumucio JP, Mendias CL. Atrogin-1, MuRF-1, and sarcopenia. Endocrine 2013; 43: 12-21.
25. Bonaldo P, Sandri M. Cellular and molecular mechanisms of muscle atrophy. Dis Model Mech 2013; 6: 25-39.
26. Das SK, Hoefler G. The role of triglyceride lipases in cancer associated cachexia. Trends Mol Med 2013; 19: 292-301.
27. Young SG, Zechner R. Biochemistry and pathophysiology of intravascular and intracellular lipolysis. Genes Dev 2013; 27: 459-84.
28. Collins P, Bing C, McCulloch P, Williams G. Muscle UCP-3 mRNA levels are elevated in weight loss associated with gastrointestinal adenocarcinoma in humans. Br J Cancer 2002; 86: 372-5.
29. Constantinou C, Fontes de Oliveira CC, Mintzopoulos D, Busquets S, He J, Kesarwani M, et al. Nuclear magnetic resonance in conjunction with functional genomics suggests mitochondrial dysfunction in a murine model of cancer cachexia. Int J Mol Med 2011; 27: 15-24.
30. Shum AM, Mahendradatta T, Taylor RJ, Painter AB, Moore MM, Tsoli M, et al. Disruption of MEF2C signaling and loss of sarcomeric and mitochondrial integrity in cancer-induced skeletal muscle wasting. Aging (Albany NY) 2012; 4: 133-43.
31. Sherry BA, Gelin J, Fong Y, Marano M, Wei H, Cerami A, et al. Anticachectin/tumor necrosis factor-alpha antibodies attenuate development of cachexia in tumor models. FASEB J 1989; 3: 1956-62.
32. Strassmann G, Fong M, Kenney JS, Jacob CO. Evidence for the involvement of interleukin 6 in experimental cancer cachexia. J Clin Invest 1992; 89: 1681-4.
33. Bonetto A, Penna F, Minero VG, Reffo P, Bonelli G, Baccino FM, et al. Deacetylase inhibitors modulate the myostatin/follistatin axis without improving cachexia in tumor-bearing mice. Curr Cancer Drug Targets 2009; 9: 608-16.
34. Alamdari N, Aversa Z, Castillero E, Hasselgren PO. Acetylation and deacetylation--novel factors in muscle wasting. Metabolism 2013; 62: 1-11.
35. Senf SM, Sandesara PB, Reed SA, Judge AR. p300 Acetyltransferase activity differentially regulates the localization and activity of the FOXO homologues in skeletal muscle. Am J Physiol Cell Physiol 2011; 300: C1490-501.
36. Black BL, Olson EN. Transcriptional control of muscle development by myocyte enhancer factor-2 (MEF2) proteins. Annu Rev Cell Dev Biol 1998; 14: 167-96.
37. Moore-Carrasco R, Garcia-Martinez C, Busquets S, Ametller E, Barreiro E, Lopez-Soriano FJ, et al. The AP-1/CJUN signaling cascade is involved in muscle differentiation: implications in muscle wasting during cancer cachexia. FEBS Lett 2006; 580: 691-6.
38. Der-Torossian H, Wysong A, Shadfar S, Willis MS, McDunn J, Couch ME. Metabolic derangements in the gastrocnemius and the effect of Compound A therapy in a murine model of cancer cachexia. J Cachexia Sarcopenia Muscle 2013; 4: 145-55.
39. Asp ML, Tian M, Wendel AA, Belury MA. Evidence for the contribution of insulin resistance to the development of cachexia in tumor-bearing mice. Int J Cancer 2010; 126: 756-63.
40. Honors MA, Kinzig KP. The role of insulin resistance in the development of muscle wasting during cancer cachexia. J Cachexia Sarcopenia Muscle 2012; 3: 5-11.
41. Moylan JS, Reid MB. Oxidative stress, chronic disease, and muscle wasting. Muscle Nerve 2007; 35: 411-29.
42. Chu PC, Kulp SK, Chen CS. Insulin-like growth factor-I receptor is suppressed through transcriptional repression and mRNA destabilization by a novel energy restriction-mimetic agent. Carcinogenesis 2013; 34: 2694-705.

## Claims

1. HDAC inhibitor AR-42 for use in suppressing cancer-induced cachexia in a mammal with cancer, wherein the HDAC inhibitor AR-42 is administered to said mammal in an amount effective to maintain the mammal's weight compared to a mammal that does not receive the HDAC inhibitor AR-42 and, wherein AR-42 for use in suppressing cancer- induced cachexia is independent of AR-I\ 2's effects on reducing tumor load.

2. HDAC inhibitor AR-42 for use of claim 1, wherein the mammal's weight is not reduced by more than about 6% after about the first 15 days following treatment with AR-42.

3. HDAC inhibitor AR-42 for use of claim 1, wherein the cancer is selected from the group consisting of pancreatic, colon, head, neck, gastric, and esophageal.

4. HDAC inhibitor AR-42 for use of claim 1, wherein the mammal is a human.

5. HDAC inhibitor AR-42 for use or claim 1, wherein AR-42 is administered in an amount of about imgikg to about 100 mg/kg of the mammal.

6. HDAC inhibitor AR-42 for use of claim 5, wherein AR-42 is administered at least once a day.

7. HDAC inhibitor AR-42 for use of claim 6, wherein AR-42 is administered twice a day in an amount of about 50 mg/kg of the mammal.

8. HDAC inhibitor AR-42 for use of claim 7, wherein levels of IL-6 are reduced by about 56% compared to a mammal that does not receive AR-42.

9. HDAC inhibitor AR-42 for use of claim 8, wherein levels of LIF are reduced by about 88% compared to a mammal that does not receive AR-42.

10. HDAC inhibitor AR-42 for use of claim 1, wherein expression of Atrogin-1 mRNA is restored to basal levels compared to a mammal that does not receive AR-42.

11. HDAC inhibitor AR-42 for use of claim 1, wherein expression of MuRF1 mRNA is restored to basal levels compared to a mammal that does not receive AR-42.

12. HDAC inhibitor AR-42 for use of claim 1, wherein a cachexia-induced increase in IL- 6Rα mRNA levels is reduced by about 85% compared to a mammal that does not receive AR-42.

13. HDAC inhibitor AR-42 for use of claim 1, wherein a cachexia-induced loss of adipose tissue is restored compared to a mammal that does not receive AR-42.

14. HDAC inhibitor AR-42 for use of claim 1, wherein a cachexia-induced reduction in skeletal muscle fiber size is restored by AR-42 compared to a mammal that does not receive AR-42.

15. HDAC inhibitor AR-42 for use in maintaining skeletal muscle weight in a mammal having cancer, wherein AR-42 is administered to said mammal in an amount effective to maintain at least about 90% of said mammal's skeletal muscle weight over a period of time of at least fifteen days compared to a mammal that does not receive AR-42 and, wherein AR-42 for use in maintaining skeletal muscle weight is independent of AR-42's effects on reducing tumor load.

## Patentansprüche

1. HDAC-Inhibitor AR-42 zur Verwendung bei der Suppression von krebsinduzierter Kachexie in einem Säugetier mit Krebs, wobei der HDAC-Inhibitor AR-42 dem Säugetier in einer wirksamen Menge verabreicht wird, um das Gewicht des Säugetiers im Vergleich zu einem Säugetier, das den HDAC-Inhibitor AR-42 nicht erhält, zu halten, und wobei AR-42 zur Verwendung bei der Suppression von krebsinduzierter Kachexie unabhängig von den Effekten von AR-42 auf eine Reduktion der Tumorlast ist.

2. HDAC-Inhibitor AR-42 zur Verwendung gemäß Anspruch 1, wobei das Gewicht des Säugetiers nach etwa den ersten 15 Tagen im Anschluss an die Behandlung mit AR-42 nicht um mehr als etwa 6 % reduziert ist.

3. HDAC-Inhibitor AR-42 zur Verwendung gemäß Anspruch 1, wobei der Krebs aus der Gruppe ausgewählt ist, die aus Bauchspeicheldrüsenkrebs, Dickdarmkrebs, Kopfkrebs, Halskrebs, Magenkrebs und Speiseröhrenkrebs besteht.

4. HDAC-Inhibitor AR-42 zur Verwendung gemäß Anspruch 1, wobei das Säugetier ein Mensch ist.

5. HDAC-Inhibitor AR-42 zur Verwendung gemäß Anspruch 1, wobei AR-42 in einer Menge von etwa 1 mg/kg bis etwa 100 mg/kg des Säugetiers verabreicht wird.

6. HDAC-Inhibitor AR-42 zur Verwendung gemäß Anspruch 5, wobei AR-42 mindestens einmal am Tag verabreicht wird.

7. HDAC-Inhibitor AR-42 zur Verwendung gemäß Anspruch 6, wobei AR-42 zweimal am Tag in einer Menge von etwa 50 mg/kg des Säugetiers verabreicht wird.

8. HDAC-Inhibitor AR-42 zur Verwendung gemäß Anspruch 7, wobei IL-6-Spiegel im Vergleich zu einem Säugetier, das kein AR-42 erhält, um etwa 56 % reduziert sind.

9. HDAC-Inhibitor AR-42 zur Verwendung gemäß Anspruch 8, wobei LIF-Spiegel im Vergleich zu einem Säugetier, das kein AR-42 erhält, um etwa 88 % reduziert sind.

10. HDAC-Inhibitor AR-42 zur Verwendung gemäß Anspruch 1, wobei die Expression von Atrogin-1-mRNA im Vergleich zu einem Säugetier, das kein AR-42 erhält, auf ihre Basalspiegel wiederhergestellt ist.

11. HDAC-Inhibitor AR-42 zur Verwendung gemäß Anspruch 1, wobei die Expression von MuRF1-mRNA im Vergleich zu einem Säugetier, das kein AR-42 erhält, auf ihre Basalspiegel wiederhergestellt ist.

12. HDAC-Inhibitor AR-42 zur Verwendung gemäß Anspruch 1, wobei eine kachexieinduzierte Zunahme von IL-6Rα-mRNA-Spiegeln im Vergleich zu einem Säugetier, das kein AR-42 erhält, um etwa 85 % reduziert ist.

13. HDAC-Inhibitor AR-42 zur Verwendung gemäß Anspruch 1, wobei ein kachexieinduzierter Verlust von Fettgewebe im Vergleich zu einem Säugetier, das kein AR-42 erhält, wiederhergestellt ist.

14. HDAC-Inhibitor AR-42 zur Verwendung gemäß Anspruch 1, wobei eine kachexieinduzierte Reduktion der Größe von Skelettmuskelfasern im Vergleich zu einem Säugetier, das kein AR-42 erhält, durch AR-42 wiederhergestellt ist.

15. HDAC-Inhibitor AR-42 zur Verwendung beim Halten des Skelettmuskelgewichts in einem Säugetier mit Krebs, wobei AR-42 dem Säugetier in einer wirksamen Menge verabreicht wird, um mindestens etwa 90 % des Skelettmuskelgewichts des Säugetiers über einen Zeitraum von mindestens fünfzehn Tagen im Vergleich zu einem Säugetier, das kein AR-42 erhält, zu halten, und wobei AR-42 zur Verwendung beim Halten des Skelettmuskelgewichts unabhängig von den Effekten von AR-42 auf eine Reduktion der Tumorlast ist.

## Revendications

1. AR-42 inhibiteur d'HDAC pour une utilisation dans la suppression de la cachexie provoquée par un cancer chez un mammifère atteint d'un cancer, l'AR-42 inhibiteur d'HDAC étant administré audit mammifère en une quantité efficace pour maintenir le poids du mammifère comparé à un mammifère ne recevant pas d'AR-42 inhibiteur d'HDAC, et l'utilisation d'AR-42 dans la suppression de la cachexie provoquée par un cancer étant indépendante des effets d'AR-42 sur la réduction de la charge tumorale.

2. AR-42 inhibiteur d'HDAC pour l'utilisation de la revendication 1, où le poids du mammifère n'est pas réduit de plus de 6 % environ après les 15 premiers jours environ suivant le traitement avec l'AR-42.

3. AR-42 inhibiteur d'HDAC pour l'utilisation de la revendication 1, où le cancer est sélectionné dans le groupe constitué du cancer pancréatique, du cancer du côlon, du cancer de la tête, du cancer du cou, du cancer gastrique, et du cancer de l'oesophage.

4. AR-42 inhibiteur d'HDAC pour l'utilisation de la revendication 1, où le mammifère est un humain.

5. AR-42 inhibiteur d'HDAC pour l'utilisation de la revendication 1, l'AR-42 étant administré en une quantité allant d'environ 1 mg/kg à environ 100 mg/kg du mammifère.

6. AR-42 inhibiteur d'HDAC pour l'utilisation de la revendication 5, l'AR-42 étant administré au moins une fois par jour.

7. AR-42 inhibiteur d'HDAC pour l'utilisation de la revendication 6, l'AR-42 étant administré deux fois par jour en une quantité d'environ 50 mg/kg du mammifère.

8. AR-42 inhibiteur d'HDAC pour l'utilisation de la revendication 7, où des niveaux d'IL-6 sont réduits d'environ 56 % comparé à un mammifère ne recevant pas d'AR-42.

9. AR-42 inhibiteur d'HDAC pour l'utilisation de la revendication 8, où des niveaux de LIF sont réduits d'environ 88 % comparé à un mammifère ne recevant pas d'AR-42.

10. AR-42 inhibiteur d'HDAC pour l'utilisation de la revendication 1, où l'expression de l'ARNm d'Atrogin-1 est restaurée à des niveaux de base comparé à un mammifère ne recevant pas d'AR-42.

11. AR-42 inhibiteur d'HDAC pour l'utilisation de la revendication 1, où l'expression de l'ARNm de MuRF1 est restaurée à des niveaux de base comparé à un mammifère ne recevant pas d'AR-42.

12. AR-42 inhibiteur d'HDAC pour l'utilisation de la revendication 1, où une augmentation de l'ARNm d'IL-6Rα, provoquée par une cachexie, est réduite d'environ 85 % comparé à un mammifère ne recevant pas d'AR-42.

13. AR-42 inhibiteur d'HDAC pour l'utilisation de la revendication 1, où une perte de tissu adipeux, provoquée par une cachexie, est restaurée comparé à un mammifère ne recevant pas d'AR-42.

14. AR-42 inhibiteur d'HDAC pour l'utilisation de la revendication 1, où une réduction de la taille des fibres de muscles squelettiques, provoquée par une cachexie, est restaurée par l'AR-42 comparé à un mammifère ne recevant pas d'AR-42.

15. AR-42 inhibiteur d'HDAC pour une utilisation dans le maintien du poids des muscles squelettiques chez un mammifère souffrant d'un cancer, l'AR-42 étant administré audit mammifère en une quantité efficace pour maintenir au moins environ 90 % dudit poids des muscles squelettiques du mammifère sur une période de temps d'au moins quinze jours comparé à un mammifère ne recevant pas d'AR-42, et l'utilisation d'AR-42 dans le maintien du poids des muscles squelettiques étant indépendante des effets d'AR-42 sur la réduction de la charge tumorale.
